(19) **Europäisches Patentamt**
**European Patent Office**
**Office européen des brevets**

(11) **EP 3 603 516 B1**

(12) **EUROPÄISCHE PATENTSCHRIFT**

(45) Veröffentlichungstag und Bekanntmachung des Hinweises auf die Patenterteilung:
**10.06.2026 Patentblatt 2026/24**

(21) Anmeldenummer: **18187106.2**

(22) Anmeldetag: **02.08.2018**

(51) Internationale Patentklassifikation (IPC):
**A61B 6/00** (2024.01) **G21K 1/06** (2006.01)

(52) Gemeinsame Patentklassifikation (CPC):
**A61B 6/4441; G21K 1/062;** A61B 6/032;
A61B 6/4078

(54) **RÖNTGENVORRICHTUNG UND VERFAHREN ZUM BETRIEB DER RÖNTGENVORRICHTUNG**

X-RAY EQUIPMENT AND METHOD FOR OPERATING SAME

DISPOSITIF À RAYONS X ET PROCÉDÉ DE FONCTIONNEMENT DU DISPOSITIF À RAYONS X

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**05.02.2020 Patentblatt 2020/06**

(73) Patentinhaber: **Siemens Healthineers AG**
**91301 Forchheim (DE)**

(72) Erfinder:
• **WIETS, Michael**
**91094 Langensendelbach (DE)**
• **WIETS, Philipp**
**91094 Langensendelbach (DE)**

(74) Vertreter: **Siemens Healthineers**
**Patent Attorneys**
**Postfach 22 16 34**
**80506 München (DE)**

(56) Entgegenhaltungen:
DE-A1- 10 139 384    DE-A1- 10 304 852
DE-A1- 10 322 137    JP-A- H01 201 200
US-A- 4 625 323

## Beschreibung

Röntgenvorrichtungen umfassen üblicherweise eine Röntgenquelle und einen Röntgendetektor, die bei einer Röntgenbildgebung auf gegenüberliegenden Seiten eines Patienten angeordnet sind. Beispielsweise sind C-Bogen-Röntgenvorrichtungen bekannt, bei denen die Röntgenquelle und der Röntgendetektor an gegenüberliegenden Enden eines C-förmigen bzw. halbkreisförmigen Bogens (kurz "C-Bogen") angeordnet sind.

Durch diese Anordnung ist aber der zur Verfügung stehende Raum zwischen Patient und Röntgenquelle bzw. zwischen Patient und Röntgendetektor beschränkt. Dieser kleine Raum kann einen Arzt bei der Durchführung von diagnostischen und/oder therapeutischen Methoden, die mit Unterstützung einer Röntgenvorrichtung durchgeführt werden müssen, behindern. Dies ist insbesondere der Fall, wenn als Röntgendetektor ein Flachdetektor verwendet wird, oder wenn die Röntgenvorrichtung weiterhin einen Bildverstärker aufweist, da solche Einrichtungen den zur Verfügung stehenden Raum weiter beschränken. Beispiele für derartige diagnostische und/oder therapeutische Methoden finden sich unter anderem in der Kardiologie bzw. Herzchirurgie, z.B. bei einer Katheteruntersuchung des Herzens oder bei einer Transkatheter-Aortenklappen-Implantation (ein englischer Fachbegriff ist "Transcatheter Aortic Valve Implantation", kurz "TAVI"). Zur Vergrößerung des zur Verfügung stehenden Raums ist es bekannt, den Abstand zwischen Röntgenquelle und Röntgendetektor zu erhöhen (ein englischer Fachbegriff für diesen Abstand ist "Source-Image-Distance", kurz "SID"), hierdurch wird aber gleichzeitig die räumliche Auflösung verringert.

Weiterhin verwenden moderne Röntgenquellen, insbesondere Hochleistungsdrehanodenröntgenröhren mehrere Emitter, bei denen jeweils Parameter wie Röntgenspannung, Pulszeiten und/oder Röntgenstrom (ein anderer Fachbegriff für "Röntgenstrom" ist "Röhrenstrom") eingestellt werden können. Wird bei einer Röntgenaufnahme eine hohe räumliche Auflösung benötigt, wird üblicherweise ein kleiner Emitter verwendet, um einen kleinen Fokus und damit eine hohe Ortsauflösung zu erreichen. Bei gleichzeitiger Verwendung eines hohen Röntgenstroms und/oder kurzen Pulszeiten sinkt die verfügbare Lebensdauer des jeweiligen Emitters. Daher können üblicherweise bestimmte Parameterkombination an solchen Emittern nicht eingestellt werden, beispielsweise können Kombinationen eines hohen Röntgenstroms mit einer kurzen Pulsdauer bzw. einer hohen Pulsfrequenz herstellerseitig ausgeschlossen werden. Es ist daher bekannt, für solche Parameterkombinationen Emitter mit einer größeren räumlichen Ausdehnung zu verwenden, die aber zu einer kleineren räumlichen Auflösung führen.

Die DE 101 39 384 A1 offenbart ein Verfahren und ein Röntgengerät zur Erzeugung einer mittels Differenzbildverfahren generierten Aufnahme eines zu durchleuchtenden Objekts.

Die US 4 625 323 A beschreibt ein Gerät zur radiologischen Untersuchung eines Patienten, welches über eine Strahlungsquelle und einen Monochromator verfügt.

Die DE 103 04 852 A1 offenbart einen Monochromator zur Verwendung bei einer Röntgeneinrichtung sowie eine Röntgeneinrichtung mit einem solchen Monochromator.

Aus der DE 103 22 137 A1 ist ein Röntgengerät bekannt, das eine Röntgenquelle, ein oder mehrere der Röntgenquelle gegenüberliegende Röntgendetektorelemente sowie ein zwischen der Röntgenquelle und den Röntgendetektorelementen liegendes Untersuchungsvolumen umfasst.

Die JP H01 201200 A beschreibt eine Röntgenbestrahlungsvorrichtung, die von einer Röntgenquelle erzeugte Röntgenstrahlen unter Verwendung einer reflektierenden Röntgenlinse eng bündelt und ein Ziel bestrahlt, wobei ein Spiegel, der sichtbares Licht reflektiert, zwischen der Röntgenquelle und der Röntgenlinse angeordnet ist.

Es ist daher die Aufgabe der vorliegenden Erfindung, eine Röntgenvorrichtung bereitzustellen, die ohne Verschlechterung der räumlichen Auflösung einen vergrößerten Raum zwischen Untersuchungsvolumen und Röntgendetektor bzw. Röntgenquelle bereitstellt, und die es weiterhin erlaubt, ohne Verschlechterung der räumlichen Auflösung Emitter mit großer räumlicher Ausdehnung zu verwenden.

Die Aufgabe wird gelöst durch eine Röntgenvorrichtung nach Anspruch 1 sowie durch ein Verfahren zum Betrieb einer Röntgenvorrichtung nach Anspruch 12. Vorteilhafte Weiterbildungen sind in den abhängigen Ansprüchen beschrieben. Nachstehend wird die erfindungsgemäße Lösung der Aufgabe sowohl in Bezug auf die beanspruchten Vorrichtungen als auch in Bezug auf das beanspruchte Verfahren beschrieben. Hierbei erwähnte Merkmale, Vorteile oder alternative Ausführungsformen sind ebenso auch auf die anderen beanspruchten Gegenstände zu übertragen und umgekehrt. Mit anderen Worten können die gegenständlichen Ansprüche (die beispielsweise auf eine Vorrichtung gerichtet sind) auch mit den Merkmalen, die in Zusammenhang mit einem Verfahren beschrieben oder beansprucht sind, weitergebildet sein. Die entsprechenden funktionalen Merkmale des Verfahrens werden dabei durch entsprechende gegenständliche Module ausgebildet.

Die Erfindung basiert auf einer Röntgenvorrichtung umfassend eine Röntgenquelle, ausgebildet zur Erzeugung von Röntgenstrahlung, weiterhin umfassend einen Röntgendetektor, und weiterhin umfassend eine Röntgenreflexionseinheit, wobei die Röntgenreflexionseinheit dazu ausgebildet ist, von der Röntgenquelle erzeugte Röntgenstrahlung derart zu reflektieren, dass die Röntgenstrahlung auf den Röntgendetektor trifft. Insbesondere ist der Röntgendetektor zum Detektieren der Röntgenstrahlung ausgebildet.

Die Erfinder haben erkannt, dass durch die Verwendung einer Röntgenreflexionseinrichtung die Röntgenquelle nicht auf der Gerade, welche einen Untersuchungsbereich und den Röntgendetektor verbindet, platziert werden muss. Hierdurch kann ohne Verschlechterung der räumlichen Auflösung ein vergrößerter Raum zwischen Untersuchungsvolumen und Röntgendetektor bzw. Röntgenquelle bereitgestellt werden.

Nach einem weiteren Aspekt der Erfindung breitet sich die Röntgenstrahlung zwischen der Röntgenquelle und der Röntgenreflexionseinheit entlang einer ersten Richtung aus, weiterhin breitet sich die Röntgenstrahlung zwischen der Röntgenreflexionseinheit und dem Röntgendetektor entlang einer zweiten Richtung aus, wobei die erste Richtung von der zweiten Richtung verschieden ist. Die erste Richtung kann hierbei insbesondere als Einfallsrichtung bezeichnet werden, die sich entlang der ersten Richtung ausbreitende Röntgenstrahlung kann insbesondere als einfallende Strahlung oder einfallende Röntgenstrahlung bezeichnet werden. Die zweite Richtung kann hierbei insbesondere auch als Ausfallsrichtung bezeichnet werden, die sich entlang der zweiten Richtung ausbreitende Röntgenstrahlung kann insbesondere als ausfallende Strahlung oder als ausfallende Röntgenstrahlung, bzw. als reflektierte Strahlung oder als reflektierte Röntgenstrahlung bezeichnet werden. Die erste Richtung kann auch als erste Ausbreitungsrichtung bezeichnet werden, die zweite Richtung kann auch als zweite Ausbreitungsrichtung bezeichnet werden. Besonders vorteilhaft ist der Winkel zwischen der ersten Richtung und der zweiten Richtung kleiner als 90° (d.h. besonders vorteilhaft ist der Winkel zwischen der negativen ersten Richtung und der zweiten Richtung größer als 90°), besonders vorteilhaft ist der Winkel zwischen der ersten Richtung und der zweiten Richtung kleiner als 60° (d.h. besonders vorteilhaft ist der Winkel zwischen der negativen ersten Richtung und der zweiten Richtung größer als 120°), und besonders vorteilhaft ist der Winkel zwischen der ersten Richtung und der zweiten Richtung kleiner als 30° (d.h. besonders vorteilhaft ist der Winkel zwischen der negativen ersten Richtung und der zweiten Richtung größer als 150°).

Eine Richtung kann insbesondere ein dreidimensionaler Vektor, eine Gerade im dreidimensionalen Raum und/oder der Richtungsvektor einer Geraden im dreidimensionalen Raum sein. Der Winkel $\alpha$ zwischen zwei Richtungen bzw. zwei Richtungsvektoren $v_1$ und $v_2$ ist gegeben durch $\alpha = \arccos(v_1 \cdot v_2)$, wobei $v_1 \cdot v_2$ das Skalarprodukt der Vektoren $v_1$ und $v_2$ bezeichnet.

Röntgenstrahlung breitet sich entlang einer Richtung gegeben durch einen dreidimensionalen Vektor $v$ aus, wenn die Röntgenstrahlung sich innerhalb eines Drehkegels mit einem beliebigen Scheitelpunkt ausbreitet, wobei die Achse des Drehkegels eine Gerade durch den Scheitelpunkt mit dem Richtungsvektor $v$ ist, und wobei der Öffnungswinkel des Drehkegels kleiner ist als 20°, insbesondere kleiner als 5°, insbesondere kleiner als 2,5°, oder insbesondere kleiner als 1° ist. Röntgenstrahlung, die sich innerhalb eines solchen Drehkegels ausbreitet, wird auch als "fächerförmige" oder "kegelförmige" Röntgenstrahlung bezeichnet. Insbesondere wird die Schnittfläche von fächerförmiger oder kegelförmiger Röntgenstrahlung mit einer zum dreidimensionalen Vektor $v$ orthogonalen Ebene entlang der Ausbreitungsrichtung größer. Röntgenstrahlung breitet sich ebenfalls entlang einer Richtung gegeben durch einen dreidimensionalen Vektor $v$ aus, wenn sich die Röntgenstrahlen der Röntgenstrahlung parallel zur Richtung $v$ ausbreitet, insbesondere als Bündel von jeweils zur Richtung $v$ parallelen Röntgenstrahlen. Röntgenstrahlung, die sich parallel zu einer Richtung $v$ ausbreitet, wird auch als "parallele" Röntgenstrahlung bezeichnet. Insbesondere bleibt die Schnittfläche von paralleler Röntgenstrahlung mit einer zum dreidimensionalen Vektor $v$ orthogonalen Ebene entlang der Ausbreitungsrichtung näherungsweise konstant.

Die Erfinder haben erkannt, dass durch eine derartige erste und zweite Richtung der Ausbreitung ein besonders großer Raum zwischen Untersuchungsvolumen und Röntgendetektor bzw. Röntgenquelle bereitgestellt werden kann.

Nach einem weiteren Aspekt der Erfindung ist der Röntgendetektor orthogonal zur zweiten Richtung angeordnet. Ein anderes Wort für "orthogonal" ist "senkrecht". Ein Röntgendetektor ist insbesondere orthogonal zu der zweiten Richtung angeordnet, wenn eine Detektionsschicht des Röntgendetektors orthogonal zur zweiten Richtung angeordnet ist. Handelt es sich bei dem Röntgendetektor um einen Flachbilddetektor umfassend einzelne Pixel, ist der Röntgendetektor insbesondere orthogonal zur zweiten Richtung angeordnet, wenn die Pixel rasterartig bezüglich einer ersten Rasterrichtung und einer zweiten Rasterrichtung angeordnet sind, wobei sich die erste Rasterrichtung und die zweite Rasterrichtung unterscheiden, und wobei sowohl die erste Rasterrichtung als auch die zweite Rasterrichtung orthogonal zur zweiten Richtung sind.

Zwei Richtungen heißen insbesondere orthogonal, wenn sie einen Winkel zwischen 80° und 100° einschließen, insbesondere wenn sie einen Winkel zwischen 85° und 95° einschließen, insbesondere, wenn sie einen Winkel zwischen 89° und 91° einschließen, und insbesondere, wenn sie einen Winkel von 90° einschließen. Eine Ebene ist insbesondere orthogonal zu einer Richtung, wenn jeder Vektor bzw. jede Gerade in dieser Ebene orthogonal zu der Richtung ist. Zwei Richtungen heißen insbesondere parallel, wenn sie einen Winkel von weniger als 10° oder von mehr als 170° einschließen, insbesondere wenn sie einen Winkel von weniger als 5° oder mehr als 175° einschließen, insbesondere wenn sie einen Winkel von weniger als 1° oder mehr als 179° einschließen, und insbesondere, wenn sie einen Vektor von genau 0° oder genau 180° einschließen. Bezeichnet $v_1$ den dreidimensionalen Vektor der ersten Richtung und $v_2$ den dreidimensionalen Vektor der zweiten Richtung, so schließen die erste Richtung und die zweite Richtung insbesondere einen Winkel er ein mit $\varphi = \arccos(v_1 \cdot v_2 / |v_1||v_2|)$, wobei $v_1 \cdot v_2$ das Skalarprodukt der Vektoren $v_1$ und $v_2$ bezeichnet, und $|v_1|$ und $|v_2|$ den Betrag bzw. die Länge des Vektors $v_1$ bzw. $v_2$ bezeichnet.

Die Erfinder haben erkannt, dass orthogonal auf den Röntgendetektor einfallende Röntgenstrahlung besonders effizient mit dem Röntgendetektor erfasst werden kann.

Nach einem weiteren Aspekt der Erfindung ist die Röntgenreflexionseinrichtung orthogonal zu einer dritten Richtung angeordnet ist, wobei die dritte Richtung eine Winkelhalbierende der negativen ersten Richtung und der zweiten Richtung ist.

Eine dritte Richtung ist eine Winkelhalbierende der negativen ersten Richtung und der zweiten Richtung, wenn der Vektor bzw. der Richtungsvektor der dritten Richtung in der von der ersten Richtung und von der zweiten Richtung aufgespannten Ebene liegt (in anderen Worten, wenn die erste Richtung, die zweite Richtung und die dritte Richtung linear abhängig sind), und wenn die negative erste Richtung und die dritte Richtung einen Winkel einschließen, der gleich dem Winkel ist, den die zweite Richtung und die dritte Richtung einschließen. Insbesondere kann die dritte Richtung $v_3$ berechnet werden als $v_3 = -v_1/|v_1| + v_2/|v_2|$.

Die Röntgenreflexionseinrichtung ist insbesondere orthogonal zu einer dritten Richtung angeordnet, wenn eine Seite der Röntgenreflexionseinrichtung orthogonal zu der dritten Richtung angeordnet ist. Die Seite der Röntgenreflexionseinrichtung ist insbesondere dann orthogonal zu der dritten Richtung, wenn diese Seite und/oder die Röntgenreflexionseinrichtung rotationssymmetrisch bezüglich der dritten Richtung sind.

Die Erfinder haben erkannt, dass durch die Verwendung einer zur Winkelhalbierenden orthogonalen Röntgenreflexionseinrichtung ein besonders hoher Anteil der Röntgenstrahlung in die zweite Richtung reflektiert wird.

Nach einem weiteren Aspekt der Erfindung ist die Röntgenreflexionseinheit zur Fokussierung der Röntgenstrahlen ausgebildet. Die Röntgenreflexionseinrichtung ist insbesondere zur Fokussierung der Röntgenstrahlung ausgebildet, wenn die Röntgenstrahlung zwischen der Röntgenquelle und der Röntgenreflexionseinrichtung eine kegelförmige Röntgenstrahlung ist, und zwischen der Röntgenreflexionseinrichtung und dem Röntgendetektor eine parallele Röntgenstrahlung ist. Insbesondere liegt in diesem Fall der Scheitelpunkt der kegelförmigen Röntgenstrahlung ausgehend von der Röntgenreflexionseinrichtung auf der durch die negative erste Richtung vorgegebenen Seite bzw. in dem durch die negative Richtung vorgegebenen Halbraum, insbesondere innerhalb der Röntgenquelle.

Die Röntgenreflexionseinrichtung ist insbesondere weiterhin zur Fokussierung der Röntgenstrahlung ausgebildet, wenn die Röntgenstrahlung zwischen der Röntgenquelle und der Röntgenreflexionseinrichtung eine parallele Röntgenstrahlung ist, und zwischen der Röntgenreflexionseinrichtung und dem Röntgendetektor eine kegelförmige Röntgenstrahlung ist. Insbesondere liegt in diesem Fall der Scheitelpunkt der kegelförmigen Röntgenstrahlung ausgehend von der Röntgenreflexionseinrichtung auf der durch die zweite Richtung vorgegebenen Seite bzw. in dem durch die zweite Richtung vorgegebenen Halbraum.

Die Röntgenreflexionseinrichtung ist insbesondere weiterhin zur Fokussierung der Röntgenstrahlung ausgebildet, wenn die Röntgenstrahlung zwischen der Röntgenquelle und der Röntgenreflexionseinrichtung eine erste kegelförmige Röntgenstrahlung ist, und zwischen der Röntgenreflexionseinrichtung und dem Röntgendetektor eine zweite kegelförmige Röntgenstrahlung ist. Insbesondere liegt in diesem Fall der Scheitelpunkt der ersten kegelförmigen Röntgenstrahlung ausgehend von der Röntgenreflexionseinrichtung auf der durch die negative erste Richtung vorgegebenen Seite bzw. in dem durch die negative Richtung vorgegebenen Halbraum, insbesondere innerhalb der Röntgenquelle. Insbesondere liegt in diesem Fall der Scheitelpunkt der zweiten kegelförmigen Röntgenstrahlung ausgehend von der Röntgenreflexionseinrichtung auf der durch die zweite Richtung vorgegebenen Seite bzw. in dem durch die zweite Richtung vorgegebenen Halbraum.

Die Röntgenreflexionseinrichtung kann insbesondere auch dazu ausgebildet sein, Röntgenstrahlung zu fokussieren, wenn die Röntgenreflexionseinrichtung eine Blende, insbesondere eine Lochblende. Hierbei ist die Lochblende insbesondere im Strahlengang der Röntgenstrahlung entlang der zweiten Richtung angeordnet.

Die Erfinder haben erkannt, dass durch eine derartige Röntgenreflexionseinheit ohne Verschlechterung der räumlichen Bildauflösung Röntgenemitter mit großer räumlicher Ausdehnung verwendet werden können, da die von diesen ausgedehnten Emittern ausgesendete Röntgenstrahlung fokussiert werden kann. Röntgenemitter mit großer räumlicher Ausdehnung können insbesondere im Vergleich zu Röntgenemittern kleinerer Ausdehnung eine größere Röntgenleistung emittieren. Weiterhin kann durch Reflexion an der Röntgenreflexionseinheit parallele Röntgenstrahlung erzeugt werden, die für die Bildgebung besser geeignet als kegelförmige Röntgenstrahlung ist.

Weiterhin muss durch eine fokussierende Röntgenreflexionseinheit die Fokussierung der Röntgenstrahlung nicht durch die Röntgenröhre selbst erfolgen. Es ist also beispielsweise sehr kostengünstig möglich, verschiedene Fokussierungen zu erreichen, bei denen jeweils die gleiche Röntgenquelle, aber unterschiedlich stark fokussierende Röntgenreflexionseinheiten verwendet werden. Alternativ können mit diesem Aufbau unterschiedliche Fokussierungen auch dadurch erreicht werden, dass der Abstand zwischen Röntgenreflexionseinheit und Röntgendetektor verändert wird.

Nach einem weiteren Aspekt der Erfindung weist die Röntgenreflexionseinheit eine konkave Seite auf, wobei die konkave Seite dazu ausgebildet ist, von der Röntgenquelle erzeugte Röntgenstrahlung zu reflektieren. Insbesondere kann die konkave Seite parabelförmig sein. Insbesondere kann die Röntgenquelle im Scheitelpunkt der parabelförmigen Seite angeordnet sein.

Insbesondere kann die Krümmung der konkaven Seite derart ausgebildet sein, dass kegelförmige Röntgenstrahlung in parallele Röntgenstrahlung reflektiert wird. Insbesondere kann die konkave Seite dazu als Rotationsparabel oder als näherungsweise Rotationsparabel ausgebildet sein. Insbesondere kann die Form der konkaven Seite auch durch die Lösung einer Differentialgleichung gegeben sein. Insbesondere können nichtparallele Anteile der Röntgenstrahlung entlang der zweiten Richtung mittels einer Blende, insbesondere einer Lochblende herausgefiltert werden.

Eine Seite ist insbesondere eine konkave Seite, wenn Strecken zwischen zwei beliebigen Punkten der Seite die Röntgenreflexionseinheit nicht schneiden, bzw. die Röntgenreflexionseinheit nur am Rand schneiden. Insbesondere kann also auch eine stückweise lineare bzw. stückweise flache Seite eine konkave Seite sein. Insbesondere ist eine Seite eine konkave Seite, wenn die mathematische Beschreibung der Seite als gekrümmte Fläche im Raum konkav ist. Eine konkave Seite kann insbesondere bezüglich nur einer ersten Achse konkav sein, insbesondere wenn sie bezüglich einer zur ersten Achse orthogonalen zweiten Achse keine Krümmung aufweist. Eine konkave Seite kann aber auch insbesondere bezüglich einer ersten Achse und einer zweiten Achse konkav sein, wobei sich die erste Achse von der zweiten Achse unterscheidet. Mit anderen Worten ist die konkave Seite dann bezüglich der ersten Achse und der zweiten Achse gekrümmt, eine solche gekrümmte Seite kann auch als "toroidal" bezeichnet werden.

Die Erfinder haben erkannt, dass eine Röntgenreflexionseinheit mit einer konkaven Seite besonders gut zum Fokussieren von Röntgenstrahlung geeignet ist.

Nach einem weiteren Aspekt der Erfindung umfasst die Röntgenreflexionseinheit der Röntgenvorrichtung einen beschichteten Spiegel und/oder einen Vielschichtspiegel und/oder einen Kristallmonochromator. Insbesondere ist die Röntgenquelle hierbei dazu ausgebildet, monochromatische Röntgenstrahlung zu erzeugen. Insbesondere kann ein Kristallmonochromator dazu ausgebildet sein, Röntgenstrahlung mittels Bragg-Reflexion zu reflektieren. Hierbei können der beschichtete Spiegel, der Vielschichtspiegel und/oder der Kristallmonochromator konkav ausgebildet sein, weiterhin können der beschichtete Spiegel, der Vielschichtspiegel und/oder der Kristallmonochromator an der konkaven Seite der Röntgenreflexionseinheit angeordnet sein. Insbesondere kann der beschichtete Spiegel ein metallbeschichteter Spiegel sein.

Die Erfinder haben erkannt, dass durch die Verwendung eines beschichteten Spiegels, eines Vielschichtspiegels und/oder eines Kristallmonochromators Röntgenstrahlung besonders effizient reflektiert werden kann. Effiziente Reflexion heißt hierbei insbesondere, dass das Verhältnis der Intensität der Röntgenstrahlung nach der Reflexion zu der Intensität der Röntgenstrahlung vor der Reflexion besonders groß ist.

Nach einem weiteren Aspekt der Erfindung umfasst die Röntgenreflexionseinheit einen Vielschichtspiegel, wobei die Röntgenstrahlung monochromatisch ist, und wobei die Schichtdicke des Vielschichtspiegels auf die Wellenlänge der monochromatischen Röntgenstrahlung abgestimmt ist. Hierbei ist es insbesondere nicht wesentlich, dass die Röntgenstrahlung monochromatisch ist, mit anderen Worten ist dann die Schichtdicke des Vielschichtspiegels auf die Wellenlänge der Röntgenstrahlung abgestimmt. Insbesondere ist die Schichtdicke des Vielschichtspiegels gleichzeitig auf die Wellenlänge der Röntgenstrahlung und auf einen Einfallwinkel der Röntgenstrahlung abgestimmt. Insbesondere ist der Einfallwinkel der Winkel zwischen der negativen ersten Richtung und der dritten Richtung. Die Schichtdicke des Vielschichtspiegels ist auf die Wellenlänge und/oder den Einfallswinkel der Röntgenstrahlung abgestimmt, wenn die Schichtdicke, die Wellenlänge und/oder der Einfallswinkel die Bragg-Bedingung für konstruktive Interferenz erfüllen. Insbesondere wenn die Röntgenstrahlung mehrere Wellenlängen aufweist, erfüllen hierbei die Schichtdicke, die Wellenlänge der Röntgenstrahlung und/oder der Einfallswinkel die Bragg-Bedingung für konstruktive Interferenz, wenn die Schichtdicke, eine der Wellenlängen der Röntgenstrahlung und/oder der Einfallswinkel die Bragg-Bedingung für konstruktive Interferenz. Insbesondere kann die eine oder die mehreren Wellenlängen der Röntgenstrahlung zwischen 8 pm und 50 pm liegen.

Der Einfallswinkel von elektromagnetischer Strahlung, insbesondere von Röntgenstrahlung, wird hierbei als Winkel zwischen der Einfallrichtung bzw. der Ausfallrichtung und dem Lot auf die Ebene der Reflexion (bzw. dem Lot auf Röntgenreflexionseinheit bzw. dem Lot auf die Seite der Röntgenreflexionseinheit) definiert. Gleichwertig kann als Einfallswinkel der kleinste Winkel zwischen der Einfallrichtung bzw. der Ausfallrichtung und der Ebene der Reflexion (bzw. der Röntgenreflexionseinheit bzw. der Seite der Röntgenreflexionseinheit) definiert werden.

Die Erfinder haben erkannt, dass durch die Verwendung eines Vielschichtspiegels mit abgestimmter Wellenlänge die Röntgenstrahlung besonders effizient reflektiert werden kann.

Nach einem weiteren Aspekt der Erfindung ist die Röntgenquelle dazu ausgebildet, Licht im optisch sichtbaren Spektrum entlang der ersten Richtung auszustrahlen. Hierbei ist die Röntgenreflexionseinheit dazu ausgebildet, das von der Röntgenquelle ausgestrahlte Licht im optischen Spektrum zu reflektieren.

Die Röntgenquelle kann insbesondere dazu ausgebildet sein, Licht im optisch sichtbaren Spektrum entlang der ersten Ausbreitungsrichtung auszustrahlen, indem die Röntgenquelle ein Umlenksystem umfasst, wobei das Umlenksystem einen Spiegel ausgebildet zum Reflektieren von Licht im optisch sichtbaren Spektrum umfasst, wobei der Spiegel für Röntgenstrahlung transparent ist. Die Röntgenreflexionseinheit ist insbesondere dazu ausgebildet, das von der Röntgenquelle ausgestrahlte Licht im optisch sichtbaren Spektrum zu reflektieren, indem die Röntgenreflexionseinheit eine erste Reflexionseinheit und eine zweite Reflexionseinheit umfasst, wobei die erste Reflexionseinheit zum Reflektieren von Röntgenstrahlung ausgebildet ist, wobei die zweite Reflexionseinheit zum Reflektieren von Licht im optisch sichtbaren Spektrum ausgebildet ist, und wobei die zweite Reflexionseinheit transparent für Röntgenstrahlung ist.

Licht im optisch sichtbaren Spektrum ist insbesondere elektromagnetische Strahlung mit einer Wellenlänge zwischen 380nm und 780nm. Insbesondere kann es sich bei dem Licht im optisch sichtbaren Spektrum um Laserlicht handeln.

Ein Objekt ist insbesondere für Röntgenstrahlung transparent, wenn die Intensität der Röntgenstrahlung nach dem Durchlaufen des Objekts mindestens 90%, insbesondere mindestens 95%, und insbesondere mindestens 99% der Intensität der Röntgenstrahlung vor dem Durchlaufen des Objekts ist.

Die Erfinder haben erkannt, dass es durch die reflektierende Anordnung von Röntgenquelle, Röntgenreflexionseinheit und Röntgendetektor schwierig ist, die räumliche Ausbreitung der Röntgenstrahlung bzw. das Raumvolumen, welches durch die Röntgenstrahlung durchdrungen wird, zu bestimmen. Dadurch können sich Bedienpersonen deutlich leichter der Röntgenstrahlung aussetzen. Durch die zusätzliche Verwendung von Licht im optisch sichtbaren Spektrum kann der Verlauf der Röntgenstrahlung sichtbar gemacht werden, und die Sicherheit der Bedienung verbessert werden. Weiterhin kann das Licht im optisch sichtbaren Spektrum zur Positionierung eines Patienten verwendet werden. Durch die Verwendung von Umlenkeinrichtungen können eigentliche Röntgenquellen kostengünstig mit Quellen für Licht im optisch sichtbaren Medium kombiniert werden.

Nach einem weiteren Aspekt der Erfindung umfasst die Röntgenvorrichtung weiterhin eine Patientenlagerungsvorrichtung, wobei die Patientenlagerungsvorrichtung im Strahlengang der Röntgenstrahlung zwischen der Röntgenreflexionseinrichtung und dem Röntgendetektor anordenbar ist.

Die Erfinder haben erkannt, dass durch eine derartige Anordnung der Patientenliege ein besonders großer Raum zwischen Untersuchungsvolumen und Röntgendetektor bzw. Röntgenquelle bereitgestellt werden kann.

Gemäß der Erfindung sind die Röntgenreflexionseinrichtung und der Röntgendetektor simultan um eine gemeinsame erste Rotationsachse rotierbar. Insbesondere können die Röntgenreflexionseinrichtung und der Röntgendetektor jeweils an einer gemeinsamen Struktur befestigt sein, wobei die gemeinsame Struktur um die erste Rotationsachse rotierbar ist. Insbesondere können die Röntgenreflexionseinrichtung und der Röntgendetektor weiterhin gemeinsam um eine dritte Rotationsachse rotierbar sein, wobei die dritte Rotationsachse orthogonal zur ersten Rotationsachse ist. Insbesondere kann die gemeinsame Struktur um die dritte Rotationsachse rotierbar sein. Bei der gemeinsamen Struktur kann es sich insbesondere um einen C-Bogen handeln. Erfindungsgemäß ist die Röntgenquelle entlang der ersten Rotationsachse angeordnet und ist die Röntgenquelle entlang der ersten Rotationsachse verschiebbar und/oder bezüglich der ersten Rotationsachse rotierbar.

Zwei Objekte sind insbesondere simultan um eine Rotationsachse rotierbar, wenn bei einer Rotation der beiden Objekte um die Rotationsachse der relative Winkel des ersten Objekts zur Rotationsachse und des zweiten Objekts zur Rotationsachse konstant ist und/oder bleibt.

Die Erfinder haben erkannt, dass durch derartig angeordnete Röntgenreflexionseinrichtung und Röntgendetektor ein Untersuchungsvolumen aus verschiedenen Richtungen aufgenommen werden kann. Insbesondere können dadurch auch dreidimensionale und/oder vierdimensionale Bilddaten des Untersuchungsbereichs aufgenommen bzw. rekonstruiert werden.

Nach einem weiteren Aspekt der Erfindung sind die Röntgenquelle und die Röntgenreflexionseinrichtung simultan um eine gemeinsame zweite Rotationsachse rotierbar. Insbesondere können die Röntgenquelle und die Röntgenreflexionseinrichtung jeweils an einer gemeinsamen Struktur befestigt sein, wobei die gemeinsame Struktur um die zweite Rotationsachse rotierbar ist. Die erste Rotationsachse und die zweite Rotationsachse können insbesondere identisch sein, weiterhin können die erste Rotationsachse und die zweite Rotationsachse insbesondere parallel sein, weiterhin können die erste Rotationsachse und die zweite Rotationsachse genau einen gemeinsamen Punkt aufweisen (in anderen Worten, die erste Rotationsachse schneidet die zweite Rotationsachse). Es ist aber auch möglich, dass die erste Rotationsachse und die zweite Rotationsachse windschief sind.

Die Erfinder haben erkannt, dass durch derartig angeordnete Röntgenreflexionseinrichtung und Röntgenquelle ein Untersuchungsvolumen besonders effizient aus verschiedenen Richtungen aufgenommen werden kann.

Nach einem weiteren möglichen Aspekt der Erfindung sind die Röntgenreflexionseinheit und die Röntgenquelle an und/oder in einer gemeinsamen Struktur angeordnet. Gemäß der Erfindung sind die Röntgenreflexionseinheit und der Röntgendetektor an und/oder in einer gemeinsamen Struktur angeordnet. Nach einem weiteren möglichen Aspekt der Erfindung sind die Röntgenreflexionseinheit, der Röntgendetektor und die Röntgenquelle an und/oder in einer gemeinsamen Struktur angeordnet. Erfindungsgemäß ist die gemeinsame Struktur dazu ausgebildet, um mindestens eine Rotationsachse zu rotieren. Insbesondere kann es sich bei der gemeinsamen Struktur um einen C-Bogen eines C-Bogen-Röntgengeräts handeln, bei der gemeinsamen Struktur kann es sich aber auch um eine Gantry eines Computertomographen handeln.

Die Erfinder haben erkannt, dass durch die Anordnung an und/oder in einer gemeinsamen Struktur die Röntgenvorrichtung besonders stabil gegenüber äußeren insbesondere mechanischen Einflüssen ist, und dass im Vergleich zu einer Anordnung ohne gemeinsame Struktur weniger bewegliche Achsen zur Veränderung von Position und/oder Orientierung der Röntgenquelle, des Röntgendetektors und/oder der Röntgenreflexionseinheit vorgesehen werden müssen, die Röntgenquelle, der Röntgendetektor und/oder die Röntgenreflexionseinheit insbesondere also kostengünstiger produziert werden kann.

Die Erfindung betrifft weiterhin ein Verfahren zum Betrieb einer Röntgenvorrichtung nach einem der Aspekte der Erfindung. Das Verfahren zum Betrieb der Röntgenvorrichtung basiert darauf, dass ein Untersuchungsbereich empfangen wird, insbesondere mittels einer Schnittstelle eines Steuerungssystems. Das Verfahren basiert weiterhin darauf, dass eine Position und/oder eine Orientierung der Röntgenreflexionseinheit eingestellt werden, insbesondere mittels einer Recheneinheit des Steuerungssystems, sodass die von der Röntgenreflexionseinheit reflektierte Röntgenstrahlung den Untersuchungsbereich durchleuchtet. Das Verfahren basiert weiterhin darauf, dass Röntgenstrahlung mittels der Röntgenquelle erzeugt wird, und dass Röntgenstrahlung mittels des Röntgendetektors detektiert wird. Bei dem Verfahren zum Betrieb einer Röntgenvorrichtung handelt es sich insbesondere um ein computerimplementiertes Verfahren. Das Verfahren kann auch derart ausgestaltet sein, dass das Erzeugen der Röntgenstrahlung mittels der Röntgenquelle und das Detektieren der Röntgenstrahlung mittels des Röntgendetektors durch den Verfahrensschritt des Erzeugens eines Steuerbefehls zur Erzeugung von Röntgenstrahlung ersetzt werden.

Die Erfinder haben erkannt, dass dieses Verfahren besonders gut geeignet ist, um eine Röntgenvorrichtung gemäß einem Aspekt der Erfindung effizient zu betreiben.

Die Erfindung kann auch ein Verfahren zum Betrieb einer Röntgenvorrichtung nach einem Aspekt der Erfindung betreffen, wobei eine Position und/oder eine Orientierung der Röntgenreflexionseinheit der Röntgenvorrichtung derart eingestellt werden, dass die von der Röntgenreflexionseinheit reflektierte Röntgenstrahlung einen vorgegebenen Untersuchungsbereich durchleuchtet.

Nach einem weiteren Aspekt des Verfahrens zum Betrieb der Röntgenvorrichtung wird die Position und/oder die Orientierung der Röntgenreflexionseinheit derart eingestellt, dass ein Reflexionswinkel der Röntgenstrahlung einem vorgegebenen Reflexionswinkel entspricht. Hierbei ist der Reflexionswinkel der Röntgenstrahlung insbesondere der Reflexionswinkel der Röntgenstrahlung bezüglich der Röntgenreflexionseinrichtung. Insbesondere ist der Reflexionswinkel der Winkel zwischen der negativen ersten Richtung und der dritten Richtung, und/der der Winkel zwischen der zweiten Richtung und der dritten Richtung.

Insbesondere kann das Verfahren weiterhin darauf basieren, dass eine Wellenlänge der Röntgenstrahlung empfangen wird, und dass der vorgegebene Reflexionswinkel basierend auf der Wellenlänge der Röntgenstrahlung bestimmt wird. Insbesondere kann der vorgegebene Reflexionswinkel derart bestimmt werden, dass die Bragg-Bedingung erfüllt ist.

Die Erfinder haben erkannt, dass durch die Einstellung eines festen Reflexionswinkels Röntgenreflexionseinheiten in einem optimalen Reflexionswirkungsgrad betrieben werden können.

Nach einem weiteren Aspekt der Erfindung umfasst das Verfahren zum Betrieb einer Röntgenvorrichtung weiterhin ein zweites Einstellen einer Position und/oder einer Orientierung des Röntgendetektors, sodass der Röntgendetektor orthogonal zur von der Röntgenreflexionseinheit reflektierten Röntgenstrahlung angeordnet ist.

Die Erfinder haben erkannt, dass durch eine derartige Einstellung der Röntgendetektor besonders effizient Röntgenstrahlung detektieren kann, da die Effizienz eines Röntgendetektors üblicherweise bei orthogonalem Einfall am höchsten ist. Durch die höhere Detektionseffizienz des Röntgendetektors kann bei gleichbleibender Bildqualität die Strahlungsbelastung eines Patienten minimiert werden.

Die Erfindung kann auch ein Steuersystem für eine Röntgenvorrichtung nach einem Aspekt der Erfindung betreffen, umfassend

- Schnittstelle, ausgebildet zum Empfangen eines Untersuchungsbereichs, weiterhin ausgebildet zum Bereitstellen eines Steuerbefehls,
- Recheneinheit, ausgebildet zum ersten Einstellen einer Position und/oder einer Orientierung der Röntgenreflexionseinheit, sodass die von der Röntgenreflexionseinheit reflektierte Röntgenstrahlung den Untersuchungsbereich durchleuchtet, weiterhin ausgebildet zum Erzeugen des Steuerbefehls zum Auslösen von Röntgenstrahlen mittels einer Röntgenquelle.

Ein solches Steuersystem kann insbesondere dazu ausgebildet sein, die zuvor beschriebenen erfindungsgemäßen Verfahren und ihre Aspekte auszuführen. Das Steuersystem ist dazu ausgebildet, diese Verfahren und ihre Aspekte auszuführen, indem die Schnittstelle und die Recheneinheit ausgebildet sind, die entsprechenden Verfahrensschritte auszuführen. Das Steuersystem kann insbesondere ein Teil der Röntgenvorrichtung sein, insbesondere kann das Steuersystem auch zur Kommunikation mit der Röntgenvorrichtung ausgebildet sein.

Die Erfindung kann auch ein Computerprogrammprodukt mit einem Computerprogramm betreffen, welches direkt in einen Speicher eines Steuersystems ladbar ist, mit Programmabschnitten, um alle Schritte des Verfahrens zum Betrieb einer Röntgenvorrichtung und/oder seiner Aspekte auszuführen, wenn die Programmabschnitte von dem Steuersystem ausgeführt werden. Die Erfindung kann weiterhin auch ein computerlesbares Speichermedium betreffen, auf welchem von einem Steuersystem lesbare und ausführbare Programmabschnitte gespeichert sind, um alle Schritte des Verfahrens zum Betrieb einer Röntgenvorrichtung und/oder seiner Aspekte auszuführen, wenn die Programmabschnitte von dem Steuersystem ausgeführt werden.

Eine weitgehend softwaremäßige Realisierung hat den Vorteil, dass auch schon bisher verwendete Steuersystem auf einfache Weise durch ein Software-Update nachgerüstet werden können, um auf die erfindungsgemäße Weise zu arbeiten. Ein solches Computerprogrammprodukt kann neben dem Computerprogramm gegebenenfalls zusätzliche Bestandteile wie z. B. eine Dokumentation und/oder zusätzliche Komponenten, sowie Hardware-Komponenten, wie z.B. Hardware-Schlüssel (Dongles etc.) zur Nutzung der Software, umfassen.

Im Allgemeinen bezeichnet Röntgenstrahlung elektromagnetische Strahlung mit einer Wellenlänge zwischen 10 nm und 1 pm, insbesondere zwischen 5 pm und 100 pm, insbesondere zwischen 8 pm und 50 pm. Insbesondere wird Röntgenstrahlung (im Unterschied zu beispielsweise Gammastrahlung) durch hochenergetische Elektronenprozesse, insbesondere durch hochenergetische Prozesse der Elektronenhülle von Atomen erzeugt. Insbesondere sind zur Erzeugung von Röntgenstrahlung Röntgenröhren und Teilchenbeschleuniger bekannt.

Monochromatische Röntgenstrahlung bezeichnet Röntgenstrahlung mit einem engen Intensitätsspektrum. Insbesondere bezeichnet man Röntgenstrahlung als monochromatische Röntgenstrahlung mit Wellenlänge $\lambda_0$, wenn das Intensitätsspektrum um die Wellenlänge $\lambda_0$ konzentriert ist. Insbesondere bezeichnet man Röntgenstrahlung als monochromatische Röntgenstrahlung, wenn der Anteil der Röntgenintensität in einem Wellenlängenintervall um die Wellenlänge $\lambda_0$ an der gesamten Röntgenintensität größer ist als 50%, insbesondere größer als 75%, insbesondere größer als 90% ist. Hierbei ist Breite des Wellenlängenintervalls insbesondere kleiner oder gleich 40% der Wellenlänge $\lambda_0$, insbesondere kleiner oder gleich als 20% der Wellenlänge $\lambda_0$, insbesondere kleiner oder gleich 10% der Wellenlänge $\lambda_0$, insbesondere kleiner oder gleich 5% der Wellenlänge $\lambda_0$. Bezeichnet $I(\lambda)$ die Intensität der Röntgenstrahlung mit Wellenlänge $\lambda$, und $I_{int}(\lambda_{max}, \lambda_{min})$ die integrierte Intensität zwischen den Wellenlängen $\lambda_{min}$ und $\lambda_{max}$, dann heißt die Röntgenstrahlung monochromatisch mit Wellenlänge $\lambda_0$, wenn $I_{int}(\lambda_{0} - \Delta\lambda, \lambda_0 + \Delta\lambda) / I_{int}(\lambda_{max}, \lambda_{min}) < a$, wobei $\Delta\lambda = b\lambda_{o}$, insbesondere mit a = 0.5, insbesondere mit a = 0.75, insbesondere mit a = 0.9, und insbesondere mit b = 0.2, insbesondere mit b = 0.1, insbesondere mit b = 0.05.

Im Allgemeinen ist ein Monochromator ein optisches Gerät zur spektralen Isolierung einer bestimmten Wellenlänge aus einer einfallenden Menge elektromagnetischer Strahlung. Ein Kristallmonochromator ist insbesondere ein Monochromator für Röntgenstrahlung, weiterhin umfasst ein Kristallmonochromator insbesondere einen Kristall. Beim Kristallmonochromator erfolgt die spektrale Isolierung insbesondere dadurch, dass die Röntgenstrahlung an verschiedenen Gitterebenen des Kristalls reflektiert wird, und dadurch Gangunterschiede entstehen. Nur wenn die Wellenlänge der einfallenden Röntgenstrahlung die Bragg-Bedingung erfüllt, tritt eine konstruktive Interferenz auf, andernfalls tritt eine destruktive Interferenz aus. Dadurch wird die Wellenlänge, welche die Bragg-Bedingung erfüllt, spektral isoliert.

Ein Vielschichtspiegel umfasst mindestens zwei unterschiedliche Materialien, die für Röntgenstrahlung bestimmter Wellenlänge unterschiedliche Brechzahlen aufweisen (optisch dichtes Material und optisch dünnes Material). Hierbei sind die Materialien mehrfach abwechselnd schichtartig angeordnet. Die Schichtdicken sind so aufeinander abgestimmt, dass für den vorgesehenen Einfallswinkel der Gangunterschied der Wellenlänge (oder einem Vielfachen der Wellenlänge) bzw. einer der Wellenlängen (oder einem Vielfachen einer der Wellenlängen) entspricht. Es kommt dann bei der Reflexion an den optisch dichteren Schichten zu einer konstruktiven Interferenz. Verfahren zur Herstellung von solchen Vielschichtspiegeln, insbesondere auch gekrümmten Vielschichtspiegeln, sind beispielsweise aus der Patentschrift US5672211A bekannt.

Ein beschichteter Spiegel, insbesondere ein metallbeschichteter Spiegel, kann Röntgenstrahlen insbesondere bei flachen Einfallswinkeln (gemessen zwischen einfallender Röntgenstrahlung und Reflexionsfläche) reflektieren, da die Reflektivität von Oberflächen bei immer flacheren Einfallswinkeln abnimmt. Insbesondere kann bei geeigneten Brechzahlen die auftretende Totalreflexion für die Reflexion von Röntgenstrahlung verwendet werden.

Die oben beschriebenen Eigenschaften, Merkmale und Vorteile dieser Erfindung sowie die Art und Weise, wie diese erreicht werden, werden klarer und deutlicher verständlich im Zusammenhang mit der folgenden Beschreibung der Ausführungsbeispiele, die im Zusammenhang mit den Zeichnungen näher erläutert werden. Durch diese Beschreibung erfolgt keine Beschränkung der Erfindung auf diese Ausführungsbeispiele. In verschiedenen Figuren sind gleiche Komponenten mit identischen Bezugszeichen versehen. Die Figuren sind in der Regel nicht maßstäblich. Es zeigen:

Fig. 1    ein erstes Ausführungsbeispiel einer Röntgenvorrichtung,
Fig. 2    ein zweites Ausführungsbeispiel einer Röntgenvorrichtung,
Fig. 3    ein drittes Ausführungsbeispiel einer Röntgenvorrichtung,
Fig. 4    ein viertes Ausführungsbeispiel einer Röntgenvorrichtung,
Fig. 5    ein fünftes Ausführungsbeispiel einer Röntgenvorrichtung,
Fig. 6    eine Röntgenreflexionseinheit umfassend einen Vielschichtspiegel,
Fig. 7    eine Röntgenreflexionseinheit umfassend einen gekrümmten Vielschichtspiegel,
Fig. 8    eine Röntgenreflexionseinheit umfassend einen Kristallmonochromator,
Fig. 9    eine Röntgenreflexionseinheit umfassend einen gekrümmten Kristallmonochromator,

Fig. 10      eine Röntgenreflexionseinheit ausgebildet zum Reflektieren von Röntgenstrahlung und von Licht im optisch sichtbaren Spektrum,

Fig. 11      ein Ablaufdiagramm eines Verfahrens zum Betrieb einer Röntgenvorrichtung.

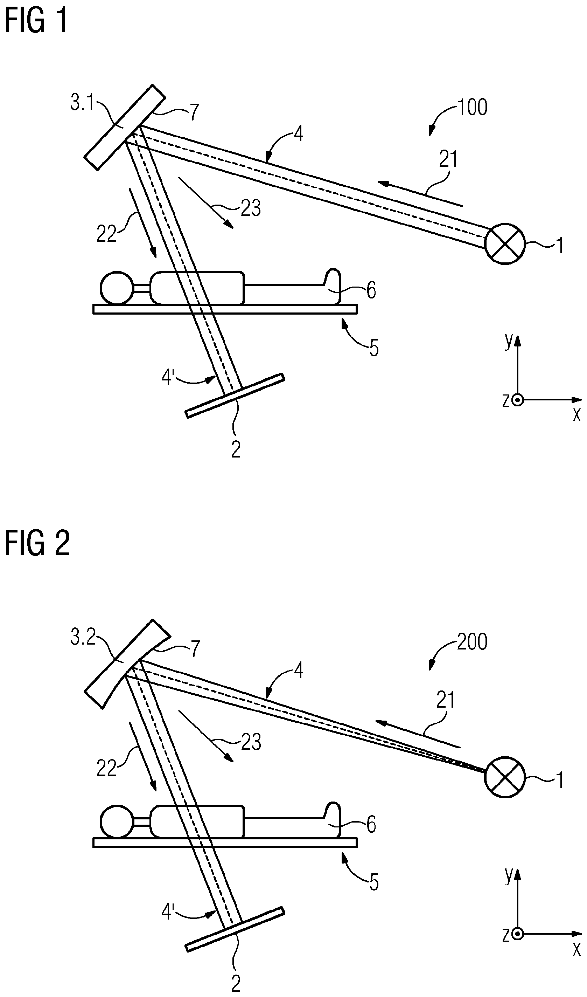

Fig. 1 zeigt ein erstes Ausführungsbeispiel einer Röntgenvorrichtung 100. Die Röntgenvorrichtung 100 umfasst eine Röntgenquelle 1, einen Röntgendetektor 2 und eine Röntgenreflexionseinheit 3.1. Die Röntgenvorrichtung umfasst in diesem Ausführungsbeispiel weiterhin eine Patientenlagerungsvorrichtung 5, auf der ein Patient 6 bzw. ein beliebiges Untersuchungsobjekt angeordnet werden kann.

Die Röntgenvorrichtung 100 befindet sich in einem Raum aufgespannt durch eine erste Koordinatenachse x, eine zweite Koordinatenachse y und eine dritte Koordinatenachse z. Die drei Koordinatenachsen x, y, z bilden ein kartesisches Rechtssystem.

Im dargestellten Ausführungsbeispiel umfasst die Röntgenquelle 1 eine Röntgenröhre, sowie vorteilhafterweise einen Monochromator und einen Kollimator. Das von der Röntgenröhre emittierte Röntgenspektrum weist zum einen Anteile durch Röntgenbremsstrahlung auf, die durch das Abbremsen der Elektronen im Kathodenmaterial der Röntgenröhre entsteht und eine breite Energieverteilung besitzt, zum anderen weist das Röntgenspektrum charakteristische Linien auf, die durch Anregen und anschließende Relaxation von Elektronen der inneren Schalen an Atomen des Kathodenmaterials entstehen. Das breite emittierte Röntgenspektrum kann durch Bragg-Reflexion am Monochromator gefiltert werden. Hierfür wird ein bekannter Kristall mit bekannten Gitterkonstanten verwendet, weiterhin werden Ein- und Ausfallswinkel der Röntgenstrahlung in der Röntgenquelle so gewählt, dass man einen Bragg-Reflex für die gewünschte Wellenlänge der monochromatischen Röntgenstrahlung erhält. Der Kollimator kann im Strahlengang der Röntgenstrahlung innerhalb der Röntgenquelle 1 vor oder nach dem Monochromator verwendet werden, um fächerförmige Röntgenstrahlung in parallele Röntgenstrahlung umzuwandeln. Als Kollimator kann insbesondere ein Parallelloch-Kollimator verwendet werden.

Alternativ kann die Röntgenquelle 1 auch keinen Monochromator aufweisen, d.h. die Röntgenquelle 1 ist in diesem Fall dazu ausgebildet, nicht-monochromatische Röntgenstrahlung bzw. polychromatische Röntgenstrahlung zu emittieren.

Alternativ zu einer Röntgenröhre kann als Röntgenquelle 1 auch ein Teilchenbeschleuniger, insbesondere zur Beschleunigung von Elektronen auf gekrümmten Bahnen, verwendet werden. Hierbei kann Röntgenstrahlung insbesondere als Synchrotronstrahlung entstehen. Zur Erzeugung von monochromatischer Röntgenstrahlung mittels Teilchenbeschleunigern sind insbesondere Undulatoren bekannt, wobei ein Undulator aus einer periodischen Anordnung von starken Magneten besteht.

Im dargestellten Ausführungsbeispiel ist die Röntgenquelle 1 dazu ausgebildet, Röntgenstrahlung 4 in eine erste Richtung 21 auszustrahlen. Mit anderen Worten breitet sich die Röntgenstrahlung 4 zwischen der Röntgenquelle 1 und der Röntgenreflexionseinheit 3.1 entlang der ersten Richtung 21 aus. Im dargestellten Ausführungsbeispiel handelt es sich bei der Röntgenstrahlung 4 insbesondere um parallele Röntgenstrahlung.

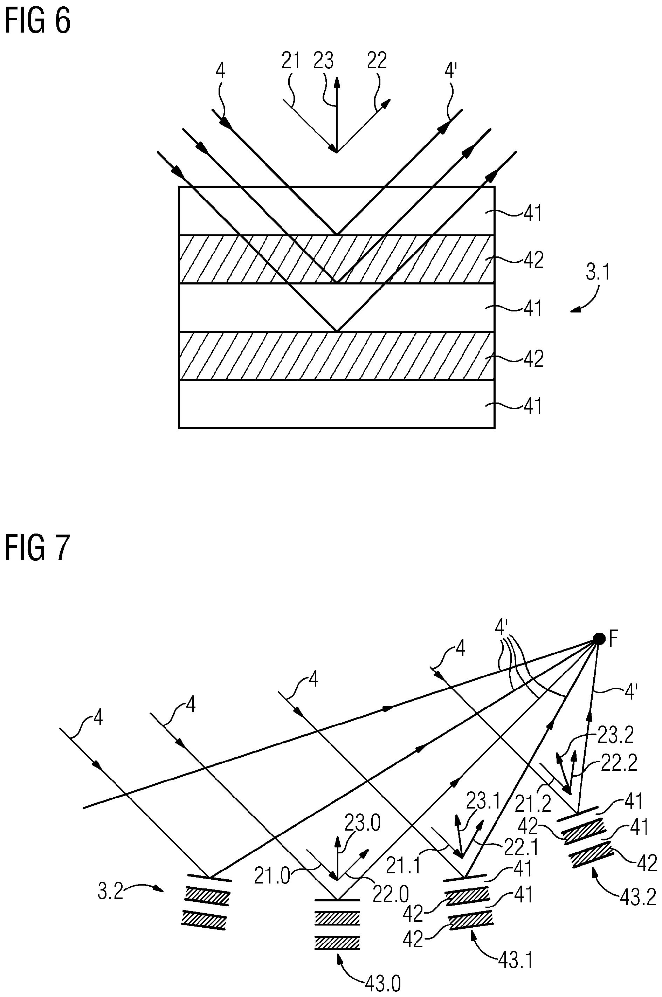

Im dargestellten Ausführungsbeispiel ist die Röntgenreflexionseinheit 3.1 dazu ausgebildet, bezüglich einer ersten Richtung 21 einfallende Röntgenstrahlung 4 derart zu reflektieren, dass sich die reflektierte Röntgenstrahlung 4' entlang einer zweiten Richtung 22 ausbreitet. Hierzu umfasst die Röntgenreflexionseinheit 3.1 einen Vielschichtspiegel, dessen Schichtdicke auf die Wellenlänge der monochromatischen Röntgenstrahlung 4 abgestimmt ist. Der genaue Aufbau einer Röntgenreflexionseinheit 3.1 mit Vielschichtspiegel ist in Fig. 6 erläutert. Der Vielschichtspiegel ist in diesem Ausführungsbeispiel an einer Seite 7 der Röntgenreflexionseinheit 3.1 angeordnet, wobei die Seite 7 der Röntgenreflexionseinheit 3.1 flach ist. Dadurch wird einfallende parallele Röntgenstrahlung 4 durch die Röntgenreflexionseinrichtung 3.1 in ausfallende parallele Röntgenstrahlung 4' reflektiert.

Die Seite 7 der Röntgenreflexionseinheit 3.1 ist orthogonal zu einer dritten Richtung 23, wobei die dritte Richtung die Winkelhalbierende der negativen ersten Richtung 21 und der zweiten Richtung 22 ist. Ist die erste Richtung 21 durch einen Vektor $v_1$ gegeben, und die zweite Richtung 22 durch einen Vektor $v_2$ gegeben, dann kann die dritte Richtung 23 insbesondere durch den Vektor

$$v_3 = -\frac{v_1}{\|v_1\|} + \frac{v_2}{\|v_2\|}$$

gegeben sein. Hierbei bezeichnet $\|v\|$ die Norm bzw. die Länge bzw. den Betrag eines Vektors. In anderen Worten ist die dritte Richtung 23 die Normale der Seite 7 der Röntgenreflexionseinheit 3.1.

Im dargestellten Ausführungsbeispiel handelt es sich beim Röntgendetektor 2 um einen Flachbilddetektor. Der Flachbilddetektor umfasst insbesondere einen Szintillator, wobei der Szintillator dazu ausgebildet ist, Röntgenstrahlung in Licht im sichtbaren Spektrum umzuwandeln. Das Licht im sichtbaren Spektrum wird mittels einer Photodiode in elektronische Ladung umgewandelt, die beispielsweise in einem Kondensator gespeichert und/oder mit einem Transistor (insbesondere einem Dünnfilmtransistor, ein englischer Fachbegriff ist "thin-film transistor", kurz "TFT") ausgelesen werden kann. Der Kondensator und/oder der Transistor bilden in diesem Fall eine Ausleseelektronik. Dabei können insbesondere mehrere Szintillatoren und Photodioden sowie die dazugehörige Ausleseelektronik in Pixeln angeordnet sein. Alternativ können statt eines Szintillators auch Materialien verwendet werden, die Röntgenstrahlung direkt in elektrische Ladungen umwandeln (Fotoleiter, z.B. amorphes Selen). Die Ausleseelektronik kann insbesondere auch in einer integrierten Schaltung angeordnet sein.

Im dargestellten Ausführungsbeispiel ist der Röntgendetektor 2 orthogonal zur zweiten Richtung 22 angeordnet. Sind die mehreren Pixel des Röntgendetektors 2 entlang einer ersten Pixelrichtung und einer zweiten Pixelrichtung angeordnet, wobei sich die zweite Pixelrichtung von der ersten Pixelrichtung unterscheidet, so ist der Röntgendetektor 2 insbesondere orthogonal zur zweiten Richtung 22 angeordnet, wenn die erste Pixelrichtung und die zweite Pixelrichtung jeweils orthogonal zur zweiten Richtung 22 sind.

Im dargestellten Ausführungsbeispiel ist eine Patientenlagerungsvorrichtung 5 im Strahlengang zwischen der Röntgenreflexionseinheit 3.1 und dem Röntgendetektor 2 angeordnet. Bei einer Patientenlagerungsvorrichtung 5 kann es sich insbesondere um eine Patientenliege handeln. Die Patientenlagerungsvorrichtung 5 kann insbesondere bezüglich jeder der drei Koordinatenachsen x, y, z beweglich ausgebildet sein. Bei Bewegung der Patientenlagerungsvorrichtung 5 bezüglich jeder der drei Koordinatenachsen x, y, z kann insbesondere die Orientierung der Patientenlagerungsvorrichtung 5 bezüglich der drei Koordinatenachsen x, y, z konstant sein. Die Patientenlagerungsvorrichtung 5 ist insbesondere zur Lagerung eines Patienten 6 ausgebildet. Die Patientenlagerungsvorrichtung 5 ist insbesondere flächig ausgebildet, weiterhin ist die Patientenlagerungsvorrichtung 5 im Wesentlichen parallel zu der von der ersten Koordinatenachse x und von der dritten Koordinatenachse z aufgespannten Ebene.

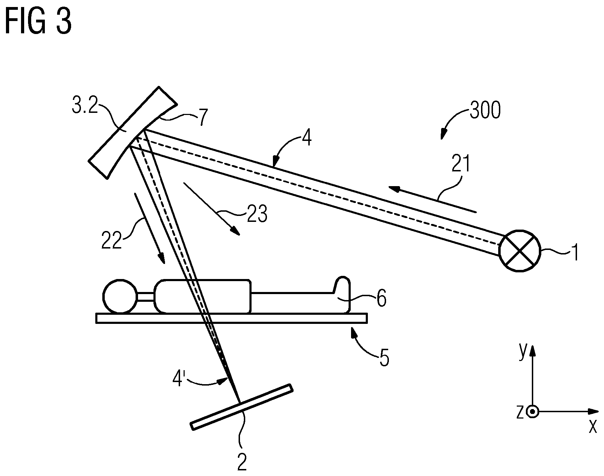

Fig. 2 und Fig. 3 zeigen ein zweites und ein drittes Ausführungsbeispiel einer Röntgenvorrichtung 200, 300. Die Röntgenvorrichtung 200, 300 umfasst hierbei eine Röntgenquelle 1, einen Röntgendetektor 2 und eine Röntgenreflexionseinrichtung 3.2, sowie eine Patientenlagerungsvorrichtung 5. Die Röntgenquelle 1, der Röntgendetektor 2, die Röntgenreflexionseinrichtung 3.2 sowie die Patientenlagerungsvorrichtung können alle vorteilhaften Aus- und Weiterbildungen umfassen, die in Bezug auf die entsprechenden Komponenten für das erste Ausführungsbeispiel beschrieben wurden.

Im Unterschied zum ersten Ausführungsbeispiel ist die Röntgenquelle 1 im zweiten Ausführungsbeispiel der Fig. 2 dazu ausgebildet, kegelförmige Röntgenstrahlung 4 zu erzeugen, wobei sich die kegelförmige Röntgenstrahlung 4 zwischen der Röntgenquelle 1 und der Röntgenreflexionseinrichtung 3.2 entlang einer ersten Richtung 21 ausbreitet. Näherungsweise kann hier die Röntgenquelle 1 als punktartige Röntgenquelle 1 aufgefasst werden, wobei die Röntgenquelle 1 dann der Scheitelpunkt der kegelförmigen Röntgenstrahlung 4 ist. Die Röntgenreflexionseinrichtung 3.2 ist hierbei weiterhin zum Fokussieren der Röntgenstrahlung 4 ausgebildet. Insbesondere ist die Röntgenreflexionseinrichtung 3.2 dazu ausgebildet, kegelförmige Röntgenstrahlung 4 in parallele Röntgenstrahlung 4' zu reflektieren. Die Röntgenreflexionseinrichtung 3.2 ist insbesondere zum Fokussieren der Röntgenstrahlung 4 ausgebildet, indem sie eine konkave Seite 7 aufweist.

Im dritten Ausführungsbeispiel ist die Röntgenreflexionseinrichtung 3.2 ebenfalls zum Fokussieren der Röntgenstrahlung 4 ausgebildet. Insbesondere ist die Röntgenreflexionseinrichtung 3.2 dazu ausgebildet, parallele Röntgenstrahlung 4 in kegelförmige Röntgenstrahlung 4' zu reflektieren, insbesondere indem sie eine konkave Seite 7 aufweist.

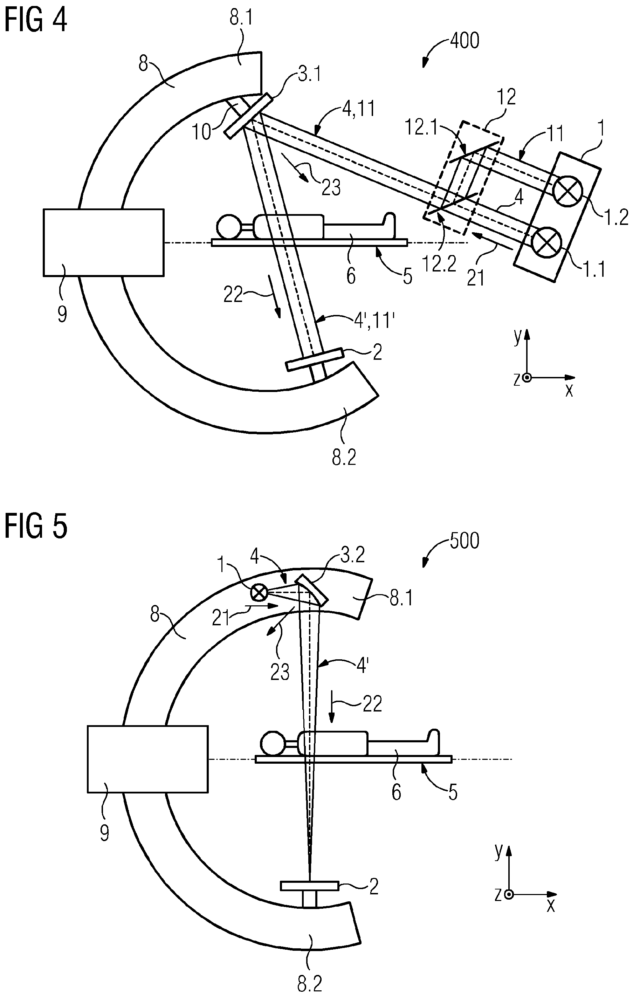

Fig. 4 und Fig. 5 zeigen ein viertes und ein fünftes Ausführungsbeispiel einer Röntgenvorrichtung 400, 500. Die Röntgenvorrichtung umfasst in diesem Ausführungsbeispiel ebenfalls eine Röntgenquelle 1, einen Röntgendetektor 2 sowie eine Röntgenreflexionseinheit 3.1, 3.2. Weiterhin sind in den Fig. 4 und Fig. 5 eine Patientenlagerungsvorrichtung 5 dargestellt, auf welcher ein Patient 6 gelagert werden kann. Die Röntgenquelle 1, der Röntgendetektor 2, die Röntgenreflexionseinrichtung 3.1, 3.2 sowie die Patientenlagerungsvorrichtung 5 können alle vorteilhaften Aus- und Weiterbildungen umfassen, die in Bezug auf die entsprechenden Komponenten für das erste Ausführungsbeispiel beschrieben wurden.

Im vierten Ausführungsbeispiel sind sowohl die Röntgenreflexionseinheit 3.1 als auch der Röntgendetektor 2 an einem C-Bogen 8 angeordnet. Insbesondere ist die Röntgenreflexionseinheit 3.1 an einem ersten Ende 8.1 des C-Bogens 8 angeordnet, und der Röntgendetektor 2 ist an einem zweiten Ende 8.2 des C-Bogens angeordnet. Hierbei befindet sich das erste Ende 8.1 und das zweite 8.2 des C-Bogens bei der Aufnahme von Röntgenbildern auf gegenüberliegenden Seiten des Patienten 6 bzw. der Patientenlagerungsvorrichtung 5. Im fünften Ausführungsbeispiel sind die Röntgenquelle 1, der Röntgendetektor 2 und die Röntgenreflexionseinheit 3.2 an einem C-Bogen angeordnet. Insbesondere sind die Röntgenquelle 1 und die Röntgenreflexionseinheit 3.2 an einem ersten Ende 8.1 des C-Bogens angeordnet, und der Röntgendetektor ist an einem zweiten Ende 8.2 des C-Bogens 8 angeordnet. Der Röntgendetektor 2 kann, insbesondere bei stationären C-Bögen, in oder gegen die zweite Richtung y verschoben werden.

Der C-Bogen 8 ist hierbei an einer Halterung 9 angeordnet. Mittels der Halterung kann der C-Bogen 8 um verschiedene Rotationsachsen rotiert werden. Alternativ zur Halterung 9 kann der Bogen auch an einem 6-Achsen Knickrobotor angeordnet sein. Beispielsweise ist der C-Bogen um eine zur dritten Koordinatenachse z parallele Rotationsachse rotierbar. Weiterhin ist der C-Bogen um eine weitere Rotationsachse parallel zu einer von der ersten Koordinatenachse x und der zweiten Koordinatenachse y aufgespannten Ebene rotierbar. Damit sind auch der Röntgendetektor 2 und die Röntgenreflexionseinheit 3.1, 3.2 um die jeweilige Rotationsachse simultan rotierbar. Weiterhin sind im fünften Ausführungsbeispiel die Röntgenquelle 1, der Röntgendetektor 2 und die Röntgenreflexionseinheit 3.1, 3.2 um die jeweilige Rotationsachse simultan rotierbar. Die räumliche Position der Halterung 9 kann verschoben werden, beispielsweise kann die Halterung 9 ihrerseits an einem beweglichen Arm mit mehreren Bewegungsachsen angeordnet sein, oder die Halterung 9 kann fahrbar ausgebildet sein.

Alternativ können auch sowohl die Röntgenreflexionseinheit 3.1 als auch der Röntgendetektor 2 an einer anderen gemeinsamen Struktur angeordnet sein, wobei die andere gemeinsame Struktur insbesondere rotierbar ausgebildet sein kann. Bei der anderen gemeinsamen Struktur kann es sich insbesondere um eine Gantry eines Computertomographen handeln.

Im vierten Ausführungsbeispiel ist die Röntgenquelle 1 weiterhin dazu ausgebildet, Licht im sichtbaren Spektrum 11 auszusenden. Hierzu umfasst die Röntgenquelle 1 eine eigentliche Röntgenquelle 1.1, die zur Erzeugung von Röntgenstrahlung 4 ausgebildet ist, und eine oder mehrere Lichtquellen 1.2, die zur Erzeugung von Licht im optisch sichtbaren Spektrum 11 ausgebildet sind. Hierbei umfasst die eigentliche Röntgenquelle 1.1 insbesondere eine Röntgenröhre, und die eine oder mehreren Lichtquellen 1.2 können insbesondere ein oder mehrere Laser sein.

Das Licht im sichtbaren Spektrum 11 wird dabei von der Lichtquelle 1.2 vorzugsweise ebenfalls entlang der ersten Richtung ausgesendet, aber parallel versetzt zu der Röntgenstrahlung 4. Durch ein Umlenksystem 12 kann das Licht im optischen Spektrum 11 mit der Röntgenstrahlung 4 in Deckung gebracht werden. Das Umlenksystem 12 umfasst im dargestellten vierten Ausführungsbeispiel 12 zwei Spiegel 12.1, 12.2, die zum Reflektieren von Licht im optischen Spektrum ausgebildet sind. Der Spiegel 12.2 im Strahlengang der Röntgenstrahlung 4 ist transparent für Röntgenstrahlung. Das Umlenksystem 12 kann auch Teil der Röntgenquelle 1 sein. Die Lichtquelle 1.2 sendet im dargestellten Ausführungsbeispiel Licht im optischen sichtbaren Spektrum 11 auch dann aus, wenn die eigentliche Röntgenquelle keine Röntgenstrahlung 4 erzeugt. Alternativ ist es auch möglich, dass die Lichtquelle 1.2 und die eigentliche Röntgenquelle 1.1 synchronisiert sind, d.h. die Lichtquelle 1.2 Licht im optisch sichtbaren Spektrum 11 im Wesentlichen nur dann aussendet, wenn die eigentliche Röntgenquelle 1.1 Röntgenstrahlung 4 erzeugt.

Die Röntgenreflexionseinheit 3.1 ist in diesem Ausführungsbeispiel dazu ausgebildet, Licht im optisch sichtbaren Spektrum 11 ähnlich oder gleich zur Röntgenstrahlung 4 zu reflektieren. Insbesondere wird das Licht im optisch sichtbaren Spektrum 11, welches sich entlang der ersten Richtung 21 ausbreitet, von der Röntgenreflexionseinheit 3.2 in die zweite Richtung 22 reflektiert.

Fig. 6 zeigt eine Röntgenreflexionseinheit 3.1 umfassend einen Vielschichtspiegel. Der Vielschichtspiegel reflektiert einfallende Röntgenstrahlung 4 entlang einer ersten Richtung 21 (dargestellt durch den Vektor $v_1$) in ausfallende Röntgenstrahlung 4' entlang einer zweiten Richtung 22 (dargestellt durch den Vektor $v_2$). Insbesondere ist der Vielschichtspiegel flächig ausgebildet bezüglich einer ersten und einer zweiten Ausdehnungsrichtung, wobei die erste und die zweite Ausdehnungsrichtung verschieden sind, und wobei die erste und die zweite Ausdehnungsrichtung jeweils orthogonal zu einer dritten Richtung 23 (dargestellt durch den Vektor $v_3$) sind. Die dritte Richtung 23 ist insbesondere die Winkelhalbierende der negativen ersten Richtung 21 und der zweiten Richtung 22.

Der Vielschichtspiegel besteht aus einer Mehrzahl von Schichten 41, 42, wobei die Schichten flächig bezüglich der ersten und der zweiten Ausdehnungsrichtung ausgedehnt sind. Die Schichten 41, 42 bestehen aus zwei verschiedenen Materialien mit unterschiedlicher optischer Aktivität, insbesondere mit unterschiedlicher Brechungszahl für elektromagnetische Wellen. Insbesondere wechseln sich jeweils eine erste Schicht 41 und eine zweite Schicht 42 ab. Die Ausdehnung der ersten Schichten 41 und der zweiten Schichten 42 bezüglich der dritten Richtung 23 wird als Schichtdicke d bezeichnet.

Bezeichnet $\lambda$ die Wellenlänge der einfallenden Röntgenstrahlung 4, und $\varphi$ den Winkel zwischen der dritten Richtung 23 und der negativen ersten Richtung 21 bzw. den Winkel zwischen der dritten Richtung 23 und der zweiten Richtung 22, dann wird die einfallende Röntgenstrahlung reflektiert, wenn die Bedingung $n \cdot \lambda = d \cdot \cos(\varphi)$ erfüllt ist (wobei $n \geq 1$ eine natürliche Zahl ist). Insbesondere gilt $\cos(\varphi) = v_1 \cdot v_2 / |v_1| \cdot |v_2|$, wobei $v_1 \cdot v_2$ das Skalarprodukt der Vektoren $v_1$ und $v_2$ bezeichnet.

Beispielsweise kann in dem dargestellten Ausführungsbeispiel eine Wellenlänge von $\lambda = 13{,}5$ nm gewählt werden, und die ersten Schichten 41 aus Silizium (optisch dünnes Medium) bestehen, und die zweiten Schichten 42 aus Molybdän (optisch dichtes Medium) bestehen.

Fig. 7 zeigt Röntgenreflexionseinheit 3.2 umfassend einen Vielschichtspiegel, die zum Fokussieren von Röntgenstrahlung 4, 4' ausgebildet ist. In der Fig. 7 sind hierbei mehrere Segmente 43.0, 43.1, 43.2 des Vielschichtspiegels dargestellt. Das Segment 43.0 kann sich insbesondere im Zentrum bzw. in einem radialen Symmetriezentrum des Vielschichtspiegels befinden. Die Abschnitte des Vielschichtspiegels außerhalb der Segmente 43.0, 43.1, 43.2 sind nicht dargestellt. Insbesondere kann der dargestellte Vielschichtspiegel als gekrümmt bzw. als konkav aufgefasst werden.

Der Vielschichtspiegel kann insbesondere aus einer Mehrzahl von flächig ausgebildeten Segmenten 43.0, 43.1, 43.2 bestehen, wobei die Segmente 43.0, 43.1, 43.2 im Wesentlichen keine Krümmung aufweisen. Mit anderen Worten ist der Vielschichtspiegel facettenartig aus den einzelnen Segmenten 43.0, 43.1, 43.2 aufgebaut. Die einzelnen Segmente können hierbei mit benachbarten Segmenten einen Punkt oder ein Liniensegment gemeinsam haben. Alternativ können die flächig ausgebildeten Segmente 43.0, 43.1, 43.2 auch konkav sein. Alternativ kann der Vielschichtspiegel auch gekrümmt, insbesondere stetig gekrümmt, insbesondere konkav ausgebildet sein. In diesem Fall können die Segmente 43.0, 43.1, 43.2 als infinitesimal klein angenommen werden. Die Eigenschaften der Segmente 43.0, 43.1, 43.2 (insbesondere die Orientierung der Segmente 43.0, 43.1, 43.2 sowie die Schichtdicke der jeweiligen Segmente) können insbesondere in beiden Fällen nur vom Abstand des jeweiligen Segments 43.0, 43.1, 43.2 vom radialen Symmetriezentrum des Vielschichtspiegels abhängen.

Im dargestellten Ausführungsbeispiel breitet sich die Röntgenstrahlung 4 zwischen der Röntgenquelle 1 und der Röntgenreflexionseinheit 3.2 als parallele Röntgenstrahlung entlang einer ersten Richtung 21 aus. Weiterhin breitet sich die Röntgenstrahlung 4' zwischen der Röntgenreflexionseinheit 3.2 und dem Röntgendetektor 2 als kegelförmige Röntgenstrahlung entlang einer zweiten Richtung 22 aus. Hierbei wird die Röntgenstrahlung in einem Fokuspunkt F fokussiert. Alternativ könnte sich die Röntgenstrahlung 4 zwischen der Röntgenquelle 1 und der Röntgenreflexionseinheit 3.2 auch als konkave Röntgenstrahlung entlang der ersten Richtung 21 ausbreiten, und weiterhin könnte sich die Röntgenstrahlung 4' zwischen der Röntgenreflexionseinheit 3.2 und dem Röntgendetektor 2 als kegelförmige Röntgenstrahlung entlang der zweiten Richtung 22 ausbreiten. Wiederum alternativ könnte sich die Röntgenstrahlung 4 zwischen der Röntgenquelle 1 und der Röntgenreflexionseinheit 3.2 auch als konkave Röntgenstrahlung entlang der ersten Richtung 21 ausbreiten, und weiterhin könnte sich die Röntgenstrahlung zwischen der Röntgenreflexionseinheit 3.2 und dem Röntgendetektor 2 als kegelförmige Röntgenstrahlung entlang der zweiten Richtung 22 ausbreiten.

Die Röntgenstrahlung fällt auf alle Segmente 43.0, 43.1, 43.2 bezüglich der ersten Richtung 21 ein, insbesondere ist also die segmentspezifische Einfallsrichtung 21.0, 21.1, 21.2 für alle Segmente 43.0, 43.1, 43.2 identisch zur ersten Richtung 21. Um eine Fokussierung zu erreichen, weicht die segmentspezifische Ausfallrichtung 22.0, 22.1, 22.2 aber von der zweiten Richtung 22 ab (außer im Segment 43.0 im Symmetriezentrum). Dementsprechend weicht aus das segmentspezifische Lot 23.0, 23.1, 23.2 von der dritten Richtung ab.

Insbesondere ist also der Winkel zwischen negativer Einfallrichtung 21.0, 21.1, 21.2 und dem segmentspezifischen Lot 23.0, 23.1, 23.2 bzw. der Winkel zwischen der Ausfallrichtung 22.0, 22.1, 22.2 segmentspezifisch. Bezeichnet $\varphi_i$ diesen Winkel im i-ten Segment 43.0, 43.1, 43.2, so muss die Schichtdicke $d_i$ des i-ten Segments 43.0, 43.1, 43.2 die Bragg-Bedingung $n\lambda = 2d_i \cdot \cos(\varphi_i)$ erfüllen, insbesondere ist also die Schichtdicke $d_i$ des i-ten Segments 43.0, 43.1, 43.2 abhängig vom jeweiligen Reflexionswinkel $\varphi_i$.

Die Segmente 43.0, 43.1, 43.2 können insbesondere auch infinitesimale Segmente 43.0, 43.1, 43.2 sein. In diesem Fall ist der Reflexionswinkel $\varphi(r)$ eines infinitesimalen Segments 43.0, 43.1, 43.2 eine Funktion des Abstands r dieses infinitesimalen Segments 43.0, 43.1, 43.2 vom Symmetriezentrum, und insbesondere ist auch die Schichtdicke $d(r)$ dieses infinitesimalen Segments 43.0, 43.1, 43.2 eine Funktion des Abstands r des infinitesimalen Segments 43.0, 43.1, 43.2 vom Symmetriezentrum, wobei wiederum die Bragg-Bedingung $n\lambda = 2d(r) \cdot \cos(\varphi(r))$ für jeden Radius r erfüllt sein muss. Verfahren zur Herstellung von solchen gekrümmten Vielschichtspiegeln, sind beispielsweise aus der Patentschrift US5672211A bekannt.

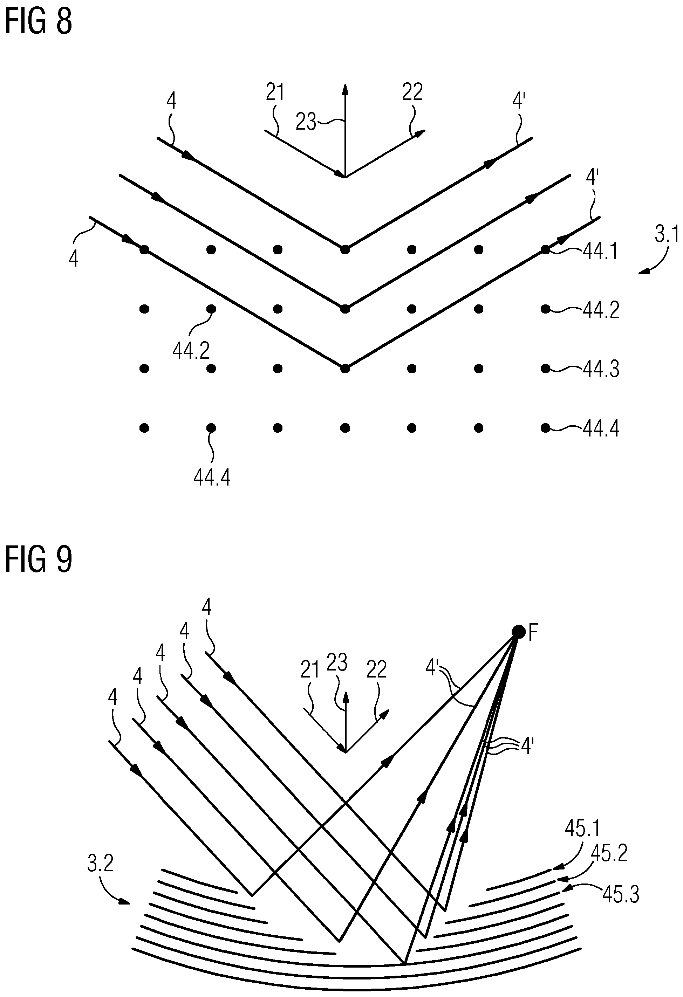

Fig. 8 zeigt eine Röntgenreflexionseinheit 3.1 umfassend einen Kristallmonochromator. Der Kristallmonochromator reflektiert einfallende Röntgenstrahlung 4 entlang einer ersten Richtung 21 (entsprechend dem Vektor $v_1$) in ausfallende Röntgenstrahlung 4' entlang einer zweiten Richtung 22 (entsprechend dem Vektor $v_2$). Insbesondere ist der Kristallmonochromator flächig ausgebildet bezüglich einer ersten und einer zweiten Ausdehnungsrichtung, wobei die erste und die zweite Ausdehnungsrichtung verschieden sind, und wobei die erste und die zweite Ausdehnungsrichtung jeweils orthogonal zu einer dritten Richtung 23 (dargestellt durch den Vektor $v_3$) sind. Die dritte Richtung 23 ist insbesondere die Winkelhalbierende der negativen ersten Richtung 21 und der zweiten Richtung 22.

Der Kristallmonochromator umfasst eine Mehrzahl von in einem Kristallgitter angeordneten Atomen 44.1, 44.2, 44.3, 44.4. Hierbei sind die Atome in bezüglich der dritten Richtung orthogonalen Schichten angeordnet, beispielsweise umfasst eine erste Schicht Atome 44.1, und eine zweite Schicht umfasst Atome 44.2, eine dritte Schicht umfasst Atome 44.3, und eine vierte Schicht umfasst Atome 44.4. Der Abstand der Schichten wird als Schichtdicke d bezeichnet. Bei den Schichten kann es sich um beliebige Schichten des Kristallmonochromators handeln, die nicht notwendigerweise die Basisvektoren des Kristallgitters enthalten. Sind die Basisvektoren des Kristallgitters $e_1$, $e_2$, $e_3$, so können prinzipiell alle Ebenen, die durch zwei linear unabhängige Ebenenvektoren aufgespannt werden, Schichten bilden, wobei jeder der linear unabhängigen Ebenenvektoren als Linearkombination $ae_1 + be_2 + ce_3$ der Basisvektoren mit ganzzahligen a, b, c dargestellt werden kann. Bei nicht-primitiven Gittern (beispielsweise kubischraumzentrierten oder kubisch-flächenzentrierten Gittern) können sogar bestimmte Linearkombinationen mit nichtganzzahligen a, b, c verwendet werden.

Im Allgemeinen kann Röntgenstrahlung 4, 4' in den Kristall eindringen, die Röntgenstrahlung wird also nicht nur an der Kristalloberfläche reflektiert, sondern an mehreren Gitterebenen des Kristallgitters. Röntgenstrahlung 4, 4', die an der äußersten Gitterebene reflektiert wird, legt hierbei eine kürzere Strecke zurück als Röntgenstrahlung, die von einer Gitterebene innerhalb des Kristalls reflektiert wird. Diese Streckendifferenz wird Gangunterschied genannt. Abhängig von diesem Gangunterschied kann es zu konstruktiver oder destruktiver Interferenz der an unterschiedlichen Gitterebenen reflektierten Röntgenstrahlung kommen.

Bezeichnet $\lambda$ die Wellenlänge der einfallenden Röntgenstrahlung 4 und $\varphi$ den Winkel zwischen der dritten Richtung 23 und der negativen ersten Richtung 21 bzw. den Winkel zwischen der dritten Richtung 23 und der zweiten Richtung 22, dann ergibt sich konstruktive Interferenz, wenn die Bedingung $n \cdot \lambda = d \cdot \cos(\varphi)$ erfüllt ist (wobei $n \geq 1$ eine natürliche Zahl ist). Insbesondere gilt $\cos(\varphi) = v_1 \cdot v_2 / |v_1| \cdot |v_2|$, wobei $v_1 \cdot v_2$ das Skalarprodukt der Vektoren $v_1$ und $v_2$ bezeichnet. In alle anderen Richtungen ergibt sich destruktive Interferenz, sodass die einfallende Röntgenstrahlung nur reflektiert wird, wenn die Bedingung $n \cdot \lambda = d \cdot \cos(\varphi)$ erfüllt ist.

Als Kristall des Kristallmonochromators kann beispielsweise ein Lithium-Fluorid-Kristall mit d = 0,201 nm verwendet werden. Weiterhin kann ein Natrium-Chlorid-Kristall mit d = 0,27 nm verwendet werden. Alternativ kann auch ein Silizium-Molybdän-Kristall mit d = 13.5 nm verwendet werden. Es sind aber natürlich noch andere Kristalle für einen Kristallmonochromator verwendbar.

Fig. 9 zeigt eine Röntgenreflexionseinheit 3.2 umfassend einen gekrümmten Kristallmonochromator, die zum Fokussieren von Röntgenstrahlung 4 ausgebildet ist. Hierbei wird parallele einfallende Röntgenstrahlung 4, wobei sich die einfallende Röntgenstrahlung bezüglich einer ersten Richtung 21 ausbreitet, in kegelförmige Röntgenstrahlung 4' mit dem Scheitelpunkt F reflektiert, wobei sich diese ausfallende Röntgenstrahlung bezüglich einer zweiten Richtung 22 ausbreitet. Der Kristallmonochromator ist hierbei rotationssymmetrisch bezüglich der dritten Richtung 23 ausgebildet, wobei die dritte Richtung 23 die Winkelhalbierende der negativen ersten Richtung 21 und der zweiten Richtung 22 ist.

Der Kristallmonochromator ist hierbei gebogen, dadurch sind auch die Kristallschichten 45.1, 45.2, 45.3 des Kristallmonochromators gebogen. Weiterhin wurde aus dem Kristallmonochromator eine konkave Auswölbung herausgefräst. Alternativ kann die konkave Auswölbung auch durch Beeinflussung des Zuchtprozesses des Kristallmonochromators erzeugt werden.

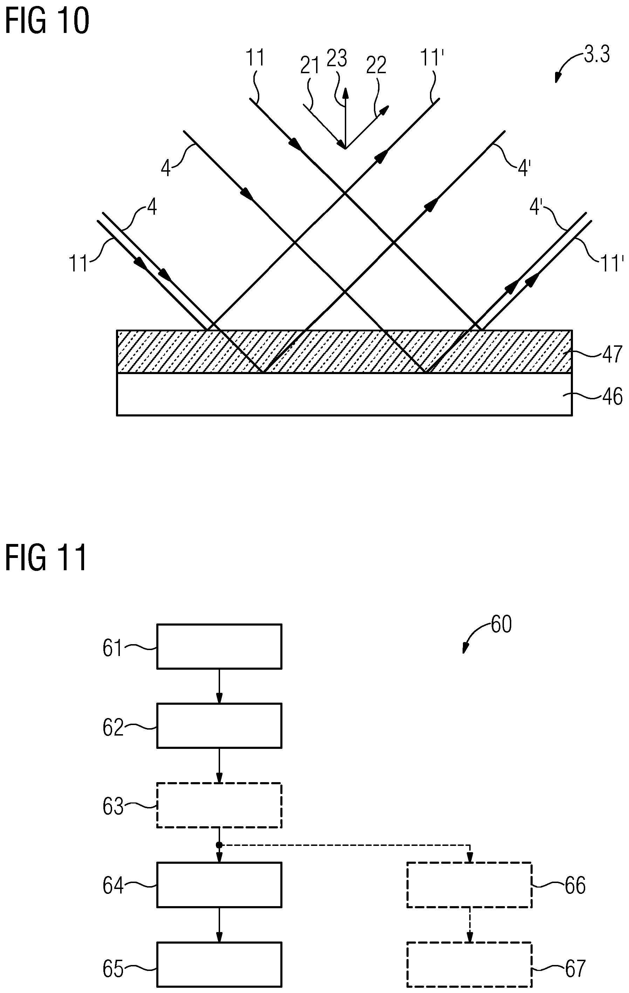

Fig. 10 zeigt eine Röntgenreflexionseinheit 3.1, die zur Reflexion von Röntgenstrahlung 4 und zur Reflexion von Licht im optisch sichtbaren Spektrum 11 ausgebildet ist. Im dargestellten Ausführungsbeispiel wird einfallende Röntgenstrahlung 4, die sich bezüglich der ersten Richtung 21 ausbreitet, in ausfallende Röntgenstrahlung 4' reflektiert, die sich bezüglich einer zweiten Richtung 22 ausbreitet. Weiterhin wird einfallendes Licht im optisch sichtbaren Spektrum 11, das sich bezüglich einer ersten Richtung 21 ausbreitet, in ausfallendes Licht im optisch sichtbaren Spektrum 11' reflektiert, das sich bezüglich der zweiten Richtung 22 ausbreitet.

Die dargestellte Röntgenreflexionseinheit 3.1 umfasst eine erste Reflexionseinheit 46 und eine zweite Reflexionseinheit 47, die bezüglich der dritten Richtung 23 übereinander angeordnet sind. Die erste Reflexionseinheit 47 ist dazu ausgebildet, Licht im optisch sichtbaren Spektrum 11, 11' zu reflektieren, und ist weiterhin transparent für Röntgenstrahlung 4, 4'. Die erste Reflexionseinheit 46 ist zum Reflektieren von Röntgenstrahlung 4, 4' ausgebildet.

Im dargestellten Ausführungsbeispiel ist die Breite und die Position des parallel einfallenden Lichts im optisch sichtbaren Spektrum 11 so gewählt, dass sowohl die einfallende Röntgenstrahlung 4 als auch die ausfallende Röntgenstrahlung 4' immer innerhalb des einfallenden Lichts im optisch sichtbaren Spektrum 11 bzw. des ausfallenden Lichts im optisch sichtbaren Spektrum 11' verläuft. Dadurch kann gewährleistet werden, dass der gesamte räumliche Strahlungsverlauf der Röntgenstrahlung 4, 4' durch das Licht im optisch sichtbaren Spektrum sichtbar gemacht wird.

Alternativ zur dargestellten bezüglich der dritten Richtung 23 übereinander angeordneten ersten Reflexionseinheit 46 und zweiten Reflexionseinheit 47 ist es auch möglich, für Röntgenstrahlung transparente Spiegel 12.1, 12.2 teilweise im Strahlengang der Röntgenstrahlung 4, 4' anzuordnen, wie es beispielsweise in Fig. 4 dargestellt ist. Durch die Spiegel 12.1, 12.2 kann Licht im optisch sichtbaren Spektrum 11 aus dem Strahlengang der einfallenden Röntgenstrahlung 4 heraus auf die erste Reflexionseinheit 46 gelenkt werden. Durch weitere Spiegel 12.1, 12.2 kann das an der ersten Reflexionseinheit reflektierte Licht im optischen Spektrum 11' wieder in den Strahlengang der reflektierten Röntgenstrahlung 4' reflektiert werden. Hierbei kann die erste Reflexionseinheit frei im Raum positioniert werden.

Fig. 11 zeigt ein Ablaufdiagramm 60 eines Ausführungsbeispiels des Verfahrens zur Bedienung einer Röntgenvorrichtung 100, 200, 300, 400, 500.

Der erste Schritt des dargestellten Ausführungsbeispiels ist das Empfangen 61 eines Untersuchungsbereichs mittels einer Schnittstelle. Im dargestellten Ausführungsbeispiel wird der Untersuchungsbereich durch gerätefeste Koordinaten relativ zur Röntgenvorrichtung 100, 200, 300, 400, 500 von einer Bedienperson eingegeben, alternativ können auch raumfeste Koordinaten verwendet werden. Alternativ kann der Untersuchungsbereich auch durch Bewegung von drehbaren Achsen der Röntgenvorrichtung 100, 200, 300, 400, 500 und/oder durch Verschiebung der Röntgenvorrichtung 100, 200, 300, 400, 500 von der Bedienperson eingestellt werden. Insbesondere ist die relative Lage des Untersuchungsbereichs zu festgelegten Teilen der Röntgenvorrichtung 100, 200, 300, 400, 500 der Bedienperson und dem Steuersystem bekannt. Insbesondere können in diesem Fall zum Empfangen REC des Untersuchungsbereichs Koordinaten der Röntgenvorrichtung 100, 200, 300, 400, 500 (insbesondere auch die Stellung von beweglichen Achsen) empfangen werden, und diese dann auf gerätefeste und/oder raumfeste Koordinaten des Untersuchungsbereichs umgerechnet werden.

Der zweite Schritt des dargestellten Ausführungsbeispiels ist das erste Einstellen 62 einer Position und/oder einer Orientierung der Röntgenreflexionseinheit 3.1, 3.2, sodass die von der Röntgenreflexionseinheit 3.1, 3.2 reflektierte Röntgenstrahlung (4') den Untersuchungsbereich durchleuchtet.

Im Folgenden wird davon ausgegangen, dass die Röntgenreflexionseinheit 3.1, 3.2 und der Röntgendetektor simultan um gemeinsame erste Rotationsachse durch den Koordinatenursprung und parallel zur dritten Koordinatenachse z drehbar sind. Weiterhin wird davon ausgegangen, dass die Röntgenreflexionseinheit 3.1, 3.2, die Röntgenquelle 1 und der Röntgendetektor 2 in einer von der ersten Koordinatenachse x und der zweiten Koordinatenachse y aufgespannten Ebene durch den Koordinatenursprung angeordnet sind. Insbesondere die Wahl der Ebene und der Rotationsachse erfolgt ohne Beschränkung der Allgemeinheit.

Die Koordinaten $x_Q$ der Röntgenquelle 1, $x_D$ des Röntgendetektors 2 sowie $x_R$ der Röntgenreflexionseinheit 3.1, 3.2 werden dann wie folgt eingeführt:

$$x_Q = \begin{pmatrix} q \\ 0 \end{pmatrix}; \quad x_D = \begin{pmatrix} 0 \\ -b \end{pmatrix}; \quad x_R = \begin{pmatrix} 0 \\ a \end{pmatrix}$$

Hierbei sind q > 0, b > 0 und a > 0 reelle Zahlen. Damit können Vektoren $v_1$ und $v_2$ bestimmt werden, welche die erste Richtung 21 und die zweite Richtung 22 darstellen bzw. bestimmen:

$$v_1 = \begin{pmatrix} -q \\ a \end{pmatrix}; \quad v_2 = \begin{pmatrix} 0 \\ -a-b \end{pmatrix}$$

Die dritte Richtung 23 kann dann als Winkelhalbierende der negative ersten Richtung 21 und der zweiten Richtung 22 bestimmt werden:

$$v_3 = \frac{1}{\sqrt{a^2+q^2}} \begin{pmatrix} q \\ -a \end{pmatrix} + \frac{1}{\sqrt{(a+b)^2}} \begin{pmatrix} 0 \\ -a-b \end{pmatrix} = \frac{1}{\sqrt{a^2+q^2}} \begin{pmatrix} q \\ -a \end{pmatrix} + \begin{pmatrix} 0 \\ -1 \end{pmatrix}$$

Beim ersten Einstellen 62 kann dann insbesondere die Orientierung der Röntgenreflexionseinheit 3.1, 3.2 derart eingestellt werden, dass die Röntgenreflexionseinheit orthogonal zur dritten Richtung 23 angeordnet ist. Die dritte Richtung ist insbesondere unabhängig vom Abstand b des Röntgendetektors 2 vom Koordinatenursprung.

Ist die Röntgenquelle 1 nicht an den Koordinaten (q, 0) angeordnet, sondern an den Koordinaten $(q_1, q_2)$, ergeben sich die die folgenden Beziehungen für die erste Richtung 21 und die zweite Richtung 22:

$$v_1 = \begin{pmatrix} -q_1 \\ a-q_2 \end{pmatrix}; \quad v_2 = \begin{pmatrix} 0 \\ -a-b \end{pmatrix}$$

Weiterhin ergibt sich somit für die dritte Richtung 23:

$$v_3 = \frac{1}{\sqrt{(a-q_2)^2+q_1^2}} \begin{pmatrix} q_1 \\ q_2-a \end{pmatrix} + \begin{pmatrix} 0 \\ -1 \end{pmatrix}$$

Sind der Röntgendetektor 2 und die Röntgenreflexionseinheit 3.1, 3.2 mit einem Winkel $\alpha$ um die gemeinsame erste Rotationsachse rotiert, ergeben sich die Koordinaten $x_D$ und $x_R$ als:

$$x_D = \begin{pmatrix} b \cdot \sin(\alpha) \\ -b \cdot \cos(\alpha) \end{pmatrix}; \quad x_R = \begin{pmatrix} -a \cdot \sin(\alpha) \\ a \cdot \cos(\alpha) \end{pmatrix}$$

Damit folgt für die erste Richtung 21 und für die zweite Richtung 22:

$$v_1 = \begin{pmatrix} -a \cdot \sin(\alpha) - q_1 \\ a \cdot \cos(\alpha) - q_2 \end{pmatrix}; \quad v_2 = \begin{pmatrix} (a+b) \cdot \sin(\alpha) \\ -(a+b) \cdot \cos(\alpha) \end{pmatrix}$$

Weiterhin ergibt sich somit für die dritte Richtung 23:

$$v_3 = \frac{1}{\sqrt{a^2+2a\left(q_1\,\sin(\alpha)-q_2\,\cos(\alpha)\right)+q_1^2+q_2^2}}\begin{pmatrix} -a\cdot\sin(\alpha)-q_1 \\ a\cdot\cos(\alpha)-q_2 \end{pmatrix} + \begin{pmatrix} \sin(\alpha) \\ -\cos(\alpha) \end{pmatrix}$$

Ein weiterer optionaler Schritt des dargestellten Ausführungsbeispiels ist das zweites Einstellen 63 einer Position und/oder einer Orientierung des Röntgendetektors 2, sodass der Röntgendetektor 2 orthogonal zur von der Röntgenreflexionseinheit 3.1, 3.2 reflektierten Röntgenstrahlung 4' angeordnet ist. In diesem Ausführungsbeispiel wird die Orientierung des Röntgendetektors derart eingestellt, dass der Röntgendetektor orthogonal zur zweiten Richtung 22 angeordnet ist, wobei die zweite Richtung 22 durch den berechneten Vektor $v_2$ gegeben ist.

Insbesondere kann alternativ auch bei vorgegebenen Koordinaten $x_D$ des Röntgendetektors 2 und $x_R$ der Röntgenreflexionseinheit 3.1, 3.2 sowie einem vorgegebenen Reflexionswinkel $\varphi$ (zwischen negativer erster Richtung 21 und dritter Richtung 23 bzw. zwischen zweiter Richtung 22 und dritter Richtung 23) die Koordinaten $x_R$ der Röntgenquelle 1 bestimmt werden. Verwendet man als vorgegebene Koordinaten

$$x_D = \begin{pmatrix} 0 \\ -b \end{pmatrix}; \quad x_R = \begin{pmatrix} 0 \\ a \end{pmatrix}$$

so ergeben sich für die dritte Richtung 23 und die erste Richtung 21 die Einheitsvektoren

$$v_3 = \begin{pmatrix} \sin(\varphi) \\ -\cos(\varphi) \end{pmatrix}; \quad v_1 = \begin{pmatrix} -\sin(2\varphi) \\ \cos(2\varphi) \end{pmatrix}.$$

Die Koordinaten $x_R$ der Röntgenquelle 1 müssen daher gewählt werden als

$$x_R = \begin{pmatrix} \sin(2\varphi) \\ a - r\cdot\cos(2\varphi) \end{pmatrix}.$$

Hierbei bezeichnet r einen frei wählbaren Abstand der Röntgenquelle 1 von der Röntgenreflexionseinheit 3.1, 3.2. Durch eine Koordinatentransformation kann gleichwertig auch die Position der Röntgenquelle 1 als bekannt vorausgesetzt werden, und die Position der Röntgenreflexionseinheit 3.1, 3.2 und/oder des Röntgendetektors 2 bestimmt werden. Die Orientierung der Röntgenreflexionseinheit 3.1, 3.2 ist hierbei jeweils durch die dritte Richtung 23 eindeutig festgelegt.

Die nächsten beiden Schritte des dargestellten Verfahrens sind das Erzeugen 64 von Röntgenstrahlung 4 mittels der Röntgenquelle 1 und das Detektieren 65 der Röntgenstrahlung 4' mittels des Röntgendetektors 2. Diese beiden Schritte können ersetzt werden durch das Bestimmen 66 einer Steueranweisung mittels der Recheneinheit, wobei die Röntgenvorrichtung 100, 200, 300, 400, 500 basierend auf der Steueranweisung die Aufnahme eines Röntgenbildes auslöst, und durch das Senden 67 der Steueranweisung an die Röntgenvorrichtung 100, 200, 300, 400, 500 mittels der Schnittstelle.

Wo noch nicht explizit geschehen, jedoch sinnvoll und im Sinne der Erfindung, können einzelne Ausführungsbeispiele, einzelne ihrer Teilaspekte oder Merkmale miteinander kombiniert bzw. ausgetauscht werden, ohne den Rahmen der hiesigen Erfindung zu verlassen. Mit Bezug zu einem Ausführungsbeispiel beschriebene Vorteile der Erfindung treffen ohne explizite Nennung, wo übertragbar, auch auf andere Ausführungsbeispiele zu.

**Patentansprüche**

**1.** Röntgenvorrichtung (100, 200, 300, 400, 500), umfassend

- eine Röntgenquelle (1), ausgebildet zur Erzeugung von Röntgenstrahlung (4),
- einen Röntgendetektor (2),
- eine Röntgenreflexionseinheit (3.1, 3.2), wobei die Röntgenreflexionseinheit (3.1, 3.2) dazu ausgebildet ist, von der Röntgenquelle (1) erzeugte Röntgenstrahlung (4) derart zu reflektieren, dass die reflektierte Röntgenstrahlung (4') auf den Röntgendetektor (2) trifft,
wobei die Röntgenreflexionseinrichtung (3.1, 3.2) und der Röntgendetektor (2) simultan um eine gemeinsame erste Rotationsachse rotierbar sind, wobei die Röntgenreflexionseinrichtung (3.1, 3.2) und der Röntgendetektor (2) an und/oder in einer gemeinsamen Struktur angeordnet sind, wobei die gemeinsame Struktur dazu ausgebildet ist, um die erste Rotationsachse zu rotieren, **dadurch gekennzeichnet, dass**
die Röntgenquelle (1) entlang der ersten Rotationsachse angeordnet ist, und dass die Röntgenquelle (1) entlang der ersten Rotationsachse verschiebbar ist und/oder bezüglich der ersten Rotationsachse rotierbar ist.

2. Röntgenvorrichtung (100, 200, 300, 400, 500) nach dem Anspruch 1, wobei sich die Röntgenstrahlung (4) zwischen der Röntgenquelle (1) und der Röntgenreflexionseinheit (3.1, 3.2) entlang einer ersten Richtung (21) ausbreitet, und wobei sich die Röntgenstrahlung (4') zwischen der Röntgenreflexionseinheit (3.1, 3.2) und dem Röntgendetektor (2) entlang einer zweiten Richtung (22) ausbreitet, wobei die erste Richtung (21) von der zweiten Richtung (22) verschieden ist.

3. Röntgenvorrichtung (100, 200, 300, 400, 500) nach dem Anspruch 2, wobei der Röntgendetektor (2) orthogonal zur zweiten Richtung (22) angeordnet ist.

4. Röntgenvorrichtung (100, 200, 300, 400, 500) nach dem Anspruch 2 oder 3, wobei die Röntgenreflexionseinrichtung (3.1, 3.2) orthogonal zu einer dritten Richtung (23) angeordnet ist, wobei die dritte Richtung (23) eine Winkelhalbierende der negativen ersten Richtung (21) und der zweiten Richtung (22) ist.

5. Röntgenvorrichtung (100, 200, 300, 400, 500) nach einem der vorstehenden Ansprüche, wobei die Röntgenreflexionseinheit (3.1, 3.2) zur Fokussierung der Röntgenstrahlen (4, 4') ausgebildet ist.

6. Röntgenvorrichtung (100, 200, 300, 400, 500) nach dem Anspruch 5, wobei die Röntgenreflexionseinheit (3.2) eine konkave Seite (7) aufweist,
wobei die konkave Seite (7) dazu ausgebildet ist, von der Röntgenquelle (1) erzeugte Röntgenstrahlung (4) zu reflektieren.

7. Röntgenvorrichtung (100, 200, 300, 400, 500) nach einem der vorstehenden Ansprüche, wobei die Röntgenreflexionseinheit einen gebogenen Kristallmonochromator umfasst, wobei der Kristallmonochromator eine konkave Auswölbung aufweist, wobei die konkave Auswölbung herausgefräst oder durch Beeinflussung des Zuchtprozesses des Kristallmonochromators erzeugt wurde.

8. Röntgenvorrichtung (100, 200, 300, 400, 500) nach einem der vorstehenden Ansprüche, wobei die Röntgenreflexionseinheit (3.1, 3.2) einen Vielschichtspiegel umfasst, wobei die von der Röntgenquelle erzeugte Röntgenstrahlung (4) und die reflektierte Röntgenstrahlung (4') monochromatisch sind, und wobei die Schichtdicke des Vielschichtspiegels auf die Wellenlänge der monochromatischen Röntgenstrahlung (4, 4') abgestimmt ist.

9. Röntgenvorrichtung (100, 200, 300, 400, 500) nach einem der vorstehenden Ansprüche, wobei die Röntgenquelle (1) weiterhin dazu ausgebildet ist, Licht im optisch sichtbaren Spektrum (11) entlang der ersten Richtung (21) auszustrahlen, wobei die Röntgenquelle (1) eine eigentliche Röntgenquelle (1.1) umfasst, wobei die eigentliche Röntgenquelle (1.1) zur Erzeugung von Röntgenstrahlung (4) ausgebildet ist,

und wobei die Röntgenquelle (1) eine oder mehrere Lichtquellen (1.2) umfasst, wobei die eine oder mehreren Lichtquellen (1.2) zur Erzeugung von Licht im optisch sichtbaren Spektrum (11) ausgebildet sind,
und wobei die Röntgenreflexionseinheit (3.1, 3.2) dazu ausgebildet ist, das von der Röntgenquelle (1) ausgestrahlte Licht im optisch sichtbaren Spektrum (11) zu reflektieren.

10. Röntgenvorrichtung (100, 200, 300, 400, 500) nach einem der vorstehenden Ansprüche, weiterhin umfassend eine Patientenlagerungsvorrichtung (5), wobei die Patientenlagerungsvorrichtung im Strahlengang der Röntgenstrahlung (4') zwischen der Röntgenreflexionseinrichtung (3.1, 3.2) und dem Röntgendetektor (2) anordenbar ist.

11. Röntgenvorrichtung nach einem der vorstehenden Ansprüche, wobei die Röntgenquelle (1) und die Röntgenreflexionseinrichtung (3.1, 3.2) simultan um eine gemeinsame zweite Rotationsachse rotierbar sind.

12. Verfahren zum Betrieb einer Röntgenvorrichtung (100, 200, 300, 400, 500) nach einem der Ansprüche 1 bis 11, umfassend:

- Empfangen (61) eines Untersuchungsbereichs,
- Erstes Einstellen (62) einer Position und/oder einer Orientierung der Röntgenreflexionseinheit (3.1, 3.2), sodass die von der Röntgenreflexionseinheit (3.1, 3.2) reflektierte Röntgenstrahlung den Untersuchungsbereich durchleuchtet,
- Erzeugen (64) von Röntgenstrahlung (4) mittels der Röntgenquelle (1), und
- Detektieren (65) der Röntgenstrahlung (4') mittels des Röntgendetektors (2).

13. Verfahren nach dem Anspruch 12, wobei die Position und/oder die Orientierung der Röntgenreflexionseinheit (3.1, 3.2) derart eingestellt werden, dass ein Reflexionswinkel der Röntgenstrahlung (4, 4') einem vorgegebenen Reflexionswinkel entspricht.

14. Verfahren nach dem Anspruch 12 oder 13, weiterhin umfassend:

- Zweites Einstellen (63) einer Position und/oder einer Orientierung des Röntgendetektors (2), sodass der Röntgendetektor (2) orthogonal zur von der Röntgenreflexionseinheit (3.1, 3.2) reflektierten Röntgenstrahlung (4') angeordnet ist.

## Claims

1. X-ray apparatus (100, 200, 300, 400, 500) including

- an X-ray source (1) embodied to generate X-rays (4),
- an X-ray detector (2),
- an X-ray reflection unit (3.1, 3.2), wherein the X-ray reflection unit (3.1, 3.2) is embodied to reflect X-rays (4) generated by the X-ray source (1) such that the reflected X-rays (4') hit the X-ray detector (2), wherein the X-ray reflection unit (3.1, 3.2) and the X-ray detector (2) can be rotated simultaneously about a common first axis of rotation, wherein the X-ray reflection unit (3.1, 3.2) and the X-ray detector (2) are arranged on and/or in a common structure, wherein the common structure is embodied to rotate about the first axis of rotation, **characterised in that** the X-ray source (1) is arranged along the first axis of rotation, and the X-ray source (1) can be shifted along the first axis of rotation and/or can be rotated with respect to the first axis of rotation.

2. X-ray apparatus (100, 200, 300, 400, 500) according to claim 1, wherein the X-rays (4) are propagated between the X-ray source (1) and the X-ray reflection unit (3.1, 3.2) along a first direction (21), and wherein the X-rays (4') are propagated between the X-ray reflection unit (3.1, 3.2) and the X-ray detector (2) along a second direction (22), wherein the first direction (21) is different from the second direction (22).

3. X-ray apparatus (100, 200, 300, 400, 500) according to claim 2, wherein the X-ray detector (2) is arranged orthogonally to the second direction (22).

4. X-ray apparatus (100, 200, 300, 400, 500) according to claim 2 or 3, wherein the X-ray reflection unit (3.1, 3.2) is arranged orthogonally to a third direction (23), wherein the third direction (23) is a bisector of the negative first direction (21) and the second direction (22).

5. X-ray apparatus (100, 200, 300, 400, 500) according to one of the preceding claims, wherein the X-ray reflection unit (3.1, 3.2) is embodied to focus the X-rays (4, 4').

6. X-ray apparatus (100, 200, 300, 400, 500) according to claim 5, wherein the X-ray reflection unit (3.2) has a concave side (7),
wherein the concave side (7) is embodied to reflect X-rays (4) generated by the X-ray source (1).

7. X-ray apparatus (100, 200, 300, 400, 500) according to one of the preceding claims, wherein the X-ray reflection unit includes a bent crystal monochromator, wherein the crystal monochromator has a concave bulge, wherein the concave bulge has been milled out or generated by influencing the growth process of the crystal monochromator.

8. X-ray apparatus (100, 200, 300, 400, 500) according to one of the preceding claims, wherein the X-ray reflection unit (3.1, 3.2) includes a multi-layer mirror, wherein the X-rays (4) generated by the X-ray source and the reflected X-rays (4') are monochromatic, and wherein the layer thickness of the multi-layer mirror is matched to the wavelength of the monochromatic X-rays (4, 4').

9. X-ray apparatus (100, 200, 300, 400, 500) according to one of the preceding claims, wherein the X-ray source (1) is further embodied to emit light in the optically visible spectrum (11) along the first direction (21), wherein the X-ray source (1) includes an actual X-ray source (1.1), wherein the actual X-ray source (1.1) is embodied to generate X-rays (4),

and wherein the X-ray source (1) includes one or more light sources (1.2), wherein the one or more light sources (1.2) are embodied to generate light in the optically visible spectrum (11), and wherein the X-ray reflection unit (3.1, 3.2) is embodied to reflect the light emitted by the X-ray source (1) in the optically visible spectrum (11).

10. X-ray apparatus (100, 200, 300, 400, 500) according to one of the preceding claims, further including a patient support apparatus (5), wherein the patient support apparatus can be arranged in the beam path of the X-rays (4') between the X-ray reflection unit (3.1, 3.2) and the X-ray detector (2).

11. X-ray apparatus according to one of the preceding claims, wherein the X-ray source (1) and the X-ray reflection unit (3.1, 3.2) can be rotated simultaneously about a common second axis of rotation.

12. Method for operating an X-ray apparatus (100, 200, 300, 400, 500) according to one of claims 1 to 11, including:

- reception (61) of an examination region,
- first setting (62) of a position and/or an orientation of the X-ray reflection unit (3.1, 3.2) so that the X-rays reflected by the X-ray reflection unit (3.1, 3.2) irradiate the examination region,
- generation (64) of X-rays (4) by means of the X-ray source (1), and
- detection (65) of the X-rays (4') by means of the X-ray detector (2).

13. Method according to claim 12, wherein the position and/or the orientation of the X-ray reflection unit (3.1, 3.2) are set such that an angle of reflection of the X-rays (4, 4') corresponds to a specified angle of reflection.

14. Method according to claim 12 or 13, further including:

- second setting (63) of a position and/or an orientation of the X-ray detector (2) so that the X-ray detector (2) is arranged orthogonally to the X-rays (4') reflected by the X-ray reflection unit (3.1, 3.2).

**Revendications**

1. Dispositif (100, 200, 300, 400, 500) à rayons X, comprenant

- une source (1) de rayons X, constituée pour la production de rayonnements (4) X,
- un détecteur (2) de rayons X,
- une unité (3.1, 3.2) de réflexion de rayons X, dans lequel l'unité (3.1, 3.2) de réflexion de rayons X est constituée pour réfléchir du rayonnement (4) X produit par la source (1) de rayons X, de manière à ce que le rayonnement (4') de rayons X réfléchi arrive sur le détecteur (2) de rayons X,

dans lequel le dispositif (3.1, 3.2) de réflexion de rayons X et le détecteur (2) de rayons X peuvent tourner simultanément autour d'un premier axe de rotation commun, dans lequel le dispositif (3.1, 3.2) de réflexion de rayons X et le détecteur (2) de rayons sont disposés sur et/ou dans une structure commune, dans lequel la structure commune est constituée pour tourner autour du premier axe de rotation, **caractérisé en ce que** la source (1) de rayons X est disposée le long du premier axe de rotation, et **en ce que** la source (1) de rayons X peut coulisser le long du premier axe de rotation et/ou peut tourner par rapport au premier axe de rotation.

2. Dispositif (100, 200, 300, 400, 500) à rayons X suivant la revendication 1, dans lequel le rayonnement (4) X se propage entre la source (1) de rayons X et l'unité (3.1, 3.2) de réflexion de rayons X suivant une première direction (21), et dans lequel le rayonnement (4') X se propage entre l'unité (3.1, 3.2) de réflexion de rayons X et le détecteur (2) de rayons X suivant une deuxième direction (22), la première direction (21) étant différente de la deuxième direction (22).

3. Dispositif (100, 200, 300, 400, 500) à rayons X suivant la revendication 2, dans lequel le détecteur (2) de rayons X est disposé orthogonalement à la deuxième direction (22).

**4.** Dispositif (100, 200, 300, 400, 500) à rayons X suivant la revendication 2 ou 3, dans lequel le dispositif (3.1, 3.2) de réflexion de rayons X est disposé orthogonalement à une troisième direction (23), dans lequel la troisième direction (23) est la bissectrice de l'angle formé entre la première direction (21) négative et la deuxième direction (22).

**5.** Dispositif (100, 200, 300, 400, 500) à rayons X suivant l'une des revendications précédentes, dans lequel l'unité (3.1, 3.2) de réflexion de rayons est constituée pour focaliser le rayonnement (4, 4') X.

**6.** Dispositif (100, 200, 300, 400, 500) à rayons X suivant la revendication 5, dans lequel l'unité (3.2) de réflexion de rayons X a une face (7) concave,
dans lequel la face (7) concave est constituée pour réfléchir du rayonnement (4) X produit par la source (1) de rayons X.

**7.** Dispositif (100, 200, 300, 400, 500) à rayons X suivant l'une des revendications précédentes, dans lequel l'unité de réflexion de rayons X comprend un monochromateur incurvé à cristal, dans lequel le monochromateur à cristal a une voussure concave, dans lequel la voussure concave est fraisée ou a été produite par influence du processus de culture du monochromateur à cristal.

**8.** Dispositif (100, 200, 300, 400, 500) à rayons X suivant l'une des revendications précédentes, dans lequel l'unité (3.1, 3.2) de réflexion de rayons X comprend un miroir à plusieurs couches, dans lequel le rayonnement (4) X produit par la source de rayons X et le rayonnement (4') de rayons X réfléchi sont monochromatiques, et dans lequel l'épaisseur de couche du miroir à plusieurs couches est adaptée à la longueur d'onde du rayonnement (4, 4') de rayons X monochromatiques.

**9.** Dispositif (100, 200, 300, 400, 500) à rayons X suivant l'une des revendications précédentes, dans lequel la source (1) de rayons X est constituée, en outre, pour émettre de la lumière dans le spectre (11) visible optiquement suivant la première direction (21), dans lequel la source (1) de rayons X comprend une source (1.1) de rayons X proprement dite, dans lequel la source (1.1) de rayons X proprement dite est constituée pour la production de rayonnement (4) X,

et dans lequel la source (1) de rayons X comprend une ou plusieurs sources (1.2) lumineuses, dans lequel la une ou les plusieurs sources (1.2) lumineuses sont constituées pour la production de lumière dans le spectre (11) visible optiquement,
et dans lequel l'unité (3.1, 3.2) de réflexion de rayons X est constituée pour réfléchir la lumière émise par la source (1) de rayons X dans le spectre (11) visible optiquement.

**10.** Dispositif (100, 200, 300, 400, 500) à rayons X suivant l'une des revendications précédentes, comprenant en outre un dispositif (5) de couchette du patient, dans lequel le dispositif de couchette du patient peut être disposé dans le trajet du rayonnement (4') X entre le dispositif (3.1, 3.2) de réflexion de rayons X et le détecteur (2) de rayons X.

**11.** Dispositif à rayons X suivant l'une des revendications précédentes, dans lequel la source (1) de rayons X et l'unité (3.1, 3.2) de réflexion de rayons X peuvent tourner simultanément autour d'un deuxième axe de rotation commun.

**12.** Procédé pour faire fonctionner un dispositif (100, 200, 300, 400, 500) de rayons X suivant l'une des revendications 1 à 11, comprenant :

- réception (61) d'une partie à examiner,
- premier réglage (62) d'une position et/ou d'une orientation de l'unité (3.1, 3.2) de réflexion de rayons X, de manière à ce que le rayonnement X réfléchi par l'unité (3.1, 3.2) de réflexion de rayons X éclaire la partie à examiner,
- production (64) de rayonnement (4) X au moyen de la source (1) de rayons X, et
- détection (65) du rayonnement (4') X au moyen du détecteur (2) de rayons X.

**13.** Procédé suivant la revendication 12, dans lequel on règle la position et/ou l'orientation de l'unité (3.1, 3.2) de réflexion de rayons X, de manière à ce qu'un angle de réflexion du rayonnement (4, 4') X corresponde à un angle de réflexion donné à l'avance.

**14.** Procédé suivant la revendication 12 ou 13, comprenant en outre :

- deuxième réglage (63) d'une position et/ou d'une orientation du détecteur (2) de rayons X, de manière à ce que le détecteur (2) de rayons X soit orthogonal au rayonnement (4') X réfléchi par l'unité (3.1, 3.2) de réflexion de rayons X.

FIG 1

FIG 2

# FIG 3

FIG 4

FIG 5

FIG 6

FIG 7

## FIG 8

## FIG 9

## FIG 10

## FIG 11

**EP 3 603 516 B1**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 10139384 A1
- US 4625323 A
- DE 10304852 A1
- DE 10322137 A1
- JP H01201200 A
- US 5672211 A